# EUROPEAN PATENT APPLICATION

(11) **EP 4 653 005 A1**
(43) Date of publication of application: **26.11.2025**
(21) Application number: 23909052.5
(22) Date of filing: 27.06.2023
(51) Int. Cl.: A61K 35/74, A61P 35/00, A61P 3/00, A61P 3/10, A61P 29/00, A61P 37/00, A61P 31/00, A61P 1/00, A61P 25/00, C12N 1/20, C12R 1/01

(54) **MICROBIAL STRAIN OF LACHNOSPIRACEAE, DRUG FOR PREVENTING OR TREATING TUMORS AND USE**

(30) Priority: 29.12.2022 CN 202211718259
(71) Applicant: Moon (Guangzhou) Biotech Co., Ltd., Guangzhou, Guangdong 510535 (CN)
(72) Inventor: ZHAO, Guozhen, Guangzhou, Guangdong 510535 (CN); XIAO, Chen, Guangzhou, Guangdong 510535 (CN); HUANG, Shaoli, Guangzhou, Guangdong 510535 (CN); KUANG, Qianwen, Guangzhou, Guangdong 510535 (CN); LIU, Zhenzhen, Guangzhou, Guangdong 510535 (CN); KONG, Ping, Guangzhou, Guangdong 510535 (CN); TAI, Lihong, Guangzhou, Guangdong 510535 (CN); ZHANG, Chenchen, Guangzhou, Guangdong 510535 (CN); LIANG, Yajun, Guangzhou, Guangdong 510535 (CN); ZHU, Ruijuan, Guangzhou, Guangdong 510535 (CN); XIAN, Yibo, Guangzhou, Guangdong 510535 (CN); ZHAO, Dongya, Guangzhou, Guangdong 510535 (CN); JIANG, Xianzhi, Guangzhou, Guangdong 510535 (CN)
(74) Representative: Prüfer & Partner mbB Patentanwälte · Rechtsanwälte
(86) International application number: PCT/CN2023/102886
(87) International publication number: WO 2024/139107

(57) **Abstract**

Provided are a microbial strain of Lachnospiraceae, a drug for preventing and/or treating at least one of tumors, metabolic diseases, diabetes, autoimmune diseases, infectious diseases, central nervous system diseases and inflammatory bowel diseases, and the use. The microbial strain of Lachnospiraceae is a new genus and species (Lachnospiraceae sp.) of the Lachnospiraceae, the nucleotide sequence of 16S rDNA is as shown in SEQ ID No. 1, and the microbial strain of the Lachnospiraceae can inhibit the growth rate of tumors and can be used for the prevention and/or treatment of tumors. The tumors include at least one of the following: liver cancer, colon cancer, rectal cancer, colorectal cancer, lung cancer, breast cancer, cervical cancer, ovarian cancer, pancreatic cancer, bile duct cancer, renal cancer and fibrosarcoma.

## Description

### TECHNICAL FIELD

The present disclosure relates to the technical field of microorganisms and uses thereof, in particular to a microbial strain of the family *Lachnospiraceae,* a drug for preventing or treating tumors, and use thereof.

### BACKGROUND ART

There are a large number of symbiotic microorganisms in the human intestinal tract. The total amount of genetic information carried by the microorganisms is 50 to 100 times that of the human genome, and known as the "gutmicorbiome", also known as the "second genome" of humans. The gutmicorbiome is the largest and most direct external environment of the human body and plays an indispensable role in maintaining human health. Up to now, there have been research reports on the role of the gutmicorbiome in nutritional disorders, metabolic abnormalities, and complex diseases (such as obesity, diabetes, inflammatory bowel disease, tumors, etc.).

In recent years, with the rapid development of molecular biology, genomics, bioinformatics analysis technology, high-throughput sequencing technology, and microbial culture technology, the role and influence of intestinal flora on intestinal and extraintestinal diseases have become increasingly clear. The intestinal flora is more like an organ with metabolic, immune and endocrine functions, which can affect the human digestive system, circulatory system and nervous system, and is closely associated with the occurrence of various human chronic diseases (such as diabetes, hypertension, cardiovascular diseases, brain diseases, etc.) and tumors. Studying the relationship between intestinal flora and human health and diseases is not only an important scientific research, but also has important significance and value in clinical diagnosis, treatment, and even translation.

Intestinal dysbacteriosis will increase the morbidity of colorectal cancer, and metabolites of some intestinal microorganisms can directly slow down carcinogenesis or inhibit tumorigenesis. Intestinal flora is implicated in breast cancer, liver cancer, etc., in addition to intestinal-related colon cancer and rectal cancer. Increasing evidence shows that intestinal microorganisms can influence tumor formation, tumor development, and tumor treatment. How to regulate the immune and inflammatory responses caused by intestinal flora so as not to induce a tumor and enhance the efficacy of anti-tumor drugs is still being explored. If these problems can be solved, it will be of great significance for future innovation and revolution in the field of tumor treatment.

Michael Scharl, et al. reported in Cell Host Microbe that *Clostridium* is associated with low tumor load, demonstrated that a mixture of symbiotic *Clostridium* strains can produce powerful anti-tumor effects through CD8⁺ T cells, and demonstrated the feasibility of using specific intestinal bacteria as monotherapy in cancer treatment by using multiple solid tumor models. CC4, a mixture of *Clostridium* strains, can exert anti-cancer effects by activating CD8⁺ T cells and downregulating immunosuppressive factors. In addition, the ability of CC4 to increase infiltration of CD8+ T cells results in tumors in a highly immunogenic state, which is beneficial to improving the response rate of CRC patients to aAnti-PD-1 therapy. These findings open a new chapter of intestinal flora supplementation as an independent therapy.

The short-chain fatty acids (SCFA) valeric acid and butyric acid can enhance the anti-tumor activities of cytotoxic T lymphocytes (CTL) and chimeric antigen receptor (CAR) T cells through metabolic and epigenetic reprogramming. Such SCFA is a rare bacterial metabolite produced by low-abundance symbionts such as *Megasphaera massiliensis,* which can be classified as a valerate-producing bacterial species. Interestingly, dominant symbiotic bacteria cannot produce valeric acid. Studies have shown that *in vitro* treatment of CTL and CAR T cells with valeric acid and butyric acid can increase the function of mTOR as a central cellular metabolism sensor and inhibit the activity of class I histone deacetylase. Such reprogramming leads to increased production of effector molecules such as CD25, IFN-γ, and TNF-α and significantly enhances the anti-tumor activities of antigen-specific CTLs and ROR1-targeting CAR T cells in syngeneic mouse melanoma and pancreatic cancer models. These experimental data reveal microbial molecules that can be used to enhance anti-tumor cellular immunity and support the identification of valeric acid and butyric acid as two SCFAs with therapeutic efficacy in cellular immunotherapy of cancers.

Therefore, further discovery, exploration, and research of microbial strains with tumor prevention or treatment potential have important application values and market prospects.

In view of this, the present disclosure is proposed.

### SUMMARY OF THE INVENTION

The objective of the present disclosure is to provide a microbial strain of *Lachnospiraceae,* a drug for preventing or treating a tumor, and the use thereof. The present inventors found that an intestinal anaerobic microbial strain of a new genus, *Lachnospiraceae sp.,* can inhibit the growth rate of tumors and can be used for the prevention and/or treatment of tumors.

The technical solutions provided by the present disclosure are as follows:
In one aspect, the present disclosure provides use of a microbial strain of *Lachnospiraceae* in the manufacture of a medicament for preventing and/or treating a tumor, wherein the microbial strain of *Lachnospiraceae* is a new species of a new genus of the family *Lachnospiraceae* (*Lachnospiraceae sp.*), the 16S rDNA of the microbial strain of *Lachnospiraceae* has a nucleotide sequence as shown in SEQ ID No. 1; and the tumor includes at least one of liver cancer, colon cancer, rectal cancer, colorectal cancer, lung cancer, breast cancer, cervical cancer, ovarian cancer, pancreatic cancer, cholangiocarcinoma, kidney cancer, and fibrosarcoma.

In one embodiment, in the use of the microbial strain of *Lachnospiraceae* in the manufacture of a medicament for preventing and/or treating a tumor, the microbial strain of *Lachnospiraceae* includes *Lachnospiraceae* MNH 46686 strain, which was deposited with the Guangdong Microbial Culture Collection Center with a deposit name of *Lachnospiraceae sp.* MNH 46686 and a deposit number of GDMCC No: 62002. The present disclosure encompasses the use of bacterial cells of the microbial strain of *Lachnospiraceae* and an extract or metabolite (such as fermentation broth) thereof in the manufacture of a medicament for preventing and/or treating a tumor and in the manufacture of a medicament for inhibiting tumor growth rate (inhibiting tumor size).

In one embodiment, the microbial strain of *Lachnospiraceae* is a new species of a new genus of the family *Lachnospiraceae* (*Lachnospiraceae sp.*), and the 16S rDNA of the microbial strain of *Lachnospiraceae* has a nucleotide sequence as shown in SEQ ID No. 1.

The strain of the present disclosure has an anti-tumor activity and can significantly inhibit tumor growth rate.

The microbial strain of *Lachnospiraceae* of the present disclosure has the following biological characteristics:
(1) Bacterial characteristics: The strain can produce spores, has no flagella, is immobile, is rod-shaped, and has a length of about 2-5 µm × 20-50 µm. The strain is Gram-negative.
(2) Colonal characteristics: After being inoculated onto an anaerobic blood plate culture medium and anaerobically cultured at 37 °C for 48 h, visible colonies were formed on the anaerobic blood plate culture medium. The colonies were round, with regular and smooth edges, about 0.5 mm in diameter, pale yellow, and opaque. No secretion was formed around the colonies.
(3) Growth characteristics and physiological and biochemical properties: The growth temperature ranges from 30 to 42 °C, and the optimal growth temperature is 37 °C. It can grow in the range of pH 7.0-9.0, and the optimal growth pH is 7.0. It cannot grow on a culture medium containing more than 2% NaCl. The MNH 46686 strain can survive and grow in a bile salt concentration ranging from 0% to 0.3%, but cannot grow when the bile salt concentration is greater than or equal to 0.4%.
(4) Other characteristics: It is resistant to erythromycin, ciprofloxacin, trimethoprim-sulfamethoxazole, ampicillin, ceftriaxone, and lincomycin; and sensitive to antibiotics such as gentamicin, chloramphenicol, tetracycline and penicillin. It can synthesize short-chain fatty acids such as acetic acid, isobutyric acid, butyric acid, and isovaleric acid in large quantities during growth.

In another aspect, the present disclosure provides a microbial agent, which includes the aforementioned microbial strain of *Lachnospiraceae* of the present disclosure or a metabolite of the microbial strain of *Lachnospiraceae.*

In another aspect, the present disclosure provides a drug for preventing and/or treating a tumor, including the aforementioned microbial strain of *Lachnospiraceae* or a metabolite of the microbial strain of *Lachnospiraceae.*

In one embodiment, the drug further includes a pharmaceutically acceptable carrier. Preferably, the carrier is selected from one or more of a diluent, dispersing agent, excipient, stabilizer, lubricant, and disintegrating agent. The excipients include mannitol, lactose, starch, microcrystalline cellulose, and the like. The disintegrating agent includes polyvinylpyrrolidone, carboxymethylcellulose, sodium carboxymethylcellulose, and the like. The lubricant includes talc, magnesium stearate, or the like.

Preferably, the drug is in the form of any one liquid formulation, solid formulation, capsule formulation, sustained-release formulation and nano-formulation. For example, the dosage form of the drug is injection, tablet, granule, pill, capsule, or the like.

In one embodiment, the composition further includes a co-drug. The co-drug includes at least one chemotherapeutic agent, photosensitizing agent, photothermal agent, and immunotherapeutic agent.

In one embodiment, the chemotherapeutic agent includes one or more of paclitaxel, camptothecin, 5-fluorouracil, cisplatin, doxorubicin, mitomycin or epirubicin;
the photosensitizing agent includes one or more of boron dipyrrole, chlorin, or Rose Bengal; and
the photothermal agent is one or more indocyanine green, new indocyanine green, or gold nanoparticle rods.

In one embodiment, the drug of the present disclosure can be used in combination with another anti-tumor drug, such as, but not limited to, oxaliplatin, mitoxantrone, adriamycin, epirubicin, etoposide, vinorelbine, methotrexate, or the like.

In one embodiment, the drug of the prevent invention can be used together with an immune adjuvant, such as CpG, IL-2, aluminum hydroxide, or the like.

In one embodiment, the immunotherapeutic agent includes an Anti-PD-1 antibody, CTLA-4 antibody, PD-L1 antibody, or PD-L1 inhibitor;
preferably, the PD-L1 inhibitor is selected from durvalumab, atezolizumab or avelumab; the Anti-PD-1 antibody or PD-L1 antibody is selected from pembrolizumab or nivolumab; and the CTLA-4 antibody is selected from ipilimumab.

In addition, the present disclosure provides a drug for preventing or treating a tumor, which includes a microbial strain of *Lachnospiraceae* and a pharmaceutically acceptable excipient, vehicle, or liquid preparation; and the microbial strain of *Lachnospiraceae* has a 16s rRNA sequence as shown in SEQ ID NO: 1. Experiments showed that the above drug has preventive and therapeutic effects on lung tumors, liver tumors, pancreatic tumors (the cell lines are KPC and Panc02), glioma (the cell line is GL261), and fibrosarcoma (the cell line is WEHI164).

Specifically, after the pancreatic cancer cell line PanO2 was inoculated subcutaneously into mice to develop tumors, an intervention with the microbial strain of *Lachnospiraceae* exhibited an effect of significantly inhibiting the growth of pancreatic tumors.

After the glioma cell line GL261 was inoculated subcutaneously into mice to develop tumors, an intervention with the microbial strain of *Lachnospiraceae* exhibited an effect of significantly inhibiting the growth of glioma.

After the fibrosarcoma cell line WEHI164 was inoculated subcutaneously into mice to develop tumors, an intervention with the microbial strain of *Lachnospiraceae* exhibited an effect of significantly inhibiting the growth of fibrosarcoma.

In addition, it should be noted that the drug for preventing or treating a tumor can also be a composition, which includes the microbial strain of *Lachnospiraceae* and a co-drug; alternatively, it includes the microbial strain of *Lachnospiraceae* and an immunosuppressive agent. The compositions obtained by combining the microbial strain of *Lachnospiraceae* as described above with the co-drug/immunosuppressive agent also have preventive and therapeutic effects on lung tumors, liver tumors, pancreatic tumors (the cell line is PanO2), glioma (the cell line is GL261), and fibrosarcoma (the cell line is WEHI164).

The co-drug includes at least one of a radiotherapeutic agent, targeting drug, chemotherapeutic agent, photosensitizing agent, photothermal agent, immunotherapeutic agent, and an agent for enhancing cell therapy, wherein:
the chemotherapeutic agent includes at least one of paclitaxel, camptothecin, 5-fluorouracil, cisplatin, doxorubicin, mitomycin or epirubicin; the photosensitizing agent includes at least one of boron dipyrrole, chlorin or Rose Bengal; and the photothermal agent includes at least one of indocyanine green, new indocyanine green or gold nanoparticle rods; and
the immunosuppressive agent includes at least one of an Anti-PD-1 antibody, CTLA-4 antibody, PD-L1 antibody, or PD-L1 inhibitor; the PD-L1 inhibitor is selected from durvalumab, atezolizumab or avelumab; the Anti-PD-1 antibody or PD-L1 antibody is selected from pembrolizumab or nivolumab; and the CTLA-4 antibody is selected from ipilimumab.

Specifically, MNH 46686 in combination with an Anti-PD-1 antibody demonstrates the effects of significantly reducing tumor volume and weight and significantly improving the response rate to tumor treatment in an ectopic syngeneic tumor model formed by subcutaneously inoculating the pancreatic cancer cell line PanO2 or KPC into mice.

MNH 46686 in combination with an Anti-PD-1 antibody demonstrates the effects of significantly reducing tumor volume and weight and significantly improving the response rate to tumor treatment in an ectopic syngeneic tumor model formed by subcutaneously inoculating the glioma cell line GL261 into mice.

MNH 46686 in combination with an Anti-PD-1 antibody demonstrates the effects of significantly reducing tumor volume and weight and significantly improving the response rate to tumor treatment in an ectopic syngeneic tumor model formed by subcutaneously inoculating the fibrosarcoma cell line WEHI164 into mice.

MNH 46686 in combination with an Anti-PD-1 antibody demonstrates the effects of significantly reducing tumor volume and weight and significantly improving the response rate to tumor treatment in an ectopic syngeneic tumor model formed by subcutaneously inoculating a liver cancer cell line (H22 or Hepal-1) into mice.

MNH 46686 in combination with an Anti-PD-1 antibody demonstrates the effects of significantly reducing tumor volume and weight and significantly improving the response rate to tumor treatment in an ectopic syngeneic tumor model formed by subcutaneously inoculating a lung cancer cell line (LLC1) into mice.

Flow cytometric analysis on combined use with Anti-PD-1 shows that the combination of the strain of the present disclosure with an Anti-PD-1 antibody significantly improves the response rate to tumor treatment, and the strain of the present disclosure used in combination with Anti-PD-1 can activate the immune system of mice, upregulate immune T cells such as CD45+CD3+, CD45+CD4+ and CD45+CD8+, increase the proportion of NK cells in peripheral blood, or the like.

As used herein, the efficacy of "enhancing" cell therapy (e.g., CAR-T) refers to a result of the administration of the composition of the prevent invention from the cell therapy (e.g., in the case of CAR-T, T cell-mediated immune response, particularly against a tumor antigen), when compared to the absence of the administration. For example, a subject treated with the composition of the present disclosure and a cell therapy can exhibit greater therapeutic effects from the cell therapy as compared to a control subject treated with the cell therapy rather than the composition of the present disclosure.

The microbial strain of *Lachnospiraceae,* a metabolite of the microbial strain of *Lachnospiraceae,* and/or supernatant of the microbial strain of *Lachnospiraceae* in the microbial strain, microbial agent, drug, and composition provided by the present disclosure can inhibit tumor growth. Preferably, inhibiting tumor growth includes at least one of inhibiting tumor volume growth, inhibiting tumor weight increase, increasing the proportion of NK cells in mouse peripheral blood to activate the mice's systemic immunity, enhancing the efficacy of an immunotherapeutic agent or CAR-T therapeutic agent, and improving tumor response rate.

The content of the microbial strain in a unit dose of the oral composition includes 2×10⁷-9×10¹⁰ CFU/mL or 1×10⁶-2×10¹⁰ CFU/mg of the microbial strain of *Lachnospiraceae* . Preferably, the unit dose is at least one of 4×10⁷ CFU/mL, 5×10⁷ CFU/mL, 6×10⁷ CFU/mL, 7×10⁷ CFU/mL, 8×10⁷ CFU/mL, 1×10⁸ CFU/mL, 2×10⁸ CFU/mL, 3×10⁸ CFU/mL, 4×10⁸ CFU/mL, 5×10⁸ CFU/mL, 6×10⁸ CFU/mL, 7×10⁸ CFU/mL, 8×10⁸ CFU/mL, 9×10⁸ CFU/mL, 1×10⁹ CFU/mL, 2×10⁹ CFU/mL, 3×10⁹ CFU/mL, 4×10⁹ CFU/mL, 5×10⁹ CFU/mL, 6×10⁹ CFU/mL, 7×10⁹ CFU/mL, 8×10⁹ CFU/mL, 9×10⁹ CFU/mL, 2×10¹⁰ or 1.2×10⁶ CFU/mg, 4×10⁶ CFU/mg, 5×10⁷ CFU/mg, 6×10⁷ CFU/mg, 7×10⁷ CFU/mg, 8×10⁷ CFU/mg, 9×10⁷ CFU/mg, 1×10⁸ CFU/mg, 2×10⁸ CFU/mg, 3×10⁸ CFU/mg, 4×10⁸ CFU/mg, 5×10⁸ CFU/mg, 6×10⁸ CFU/mg, 7×10⁸ CFU/mg, 8×10⁸ CFU/mg and 9×10⁸ CFU/mg.

The microbial strain of *Lachnospiraceae,* metabolite of the microbial strain of *Lachnospiraceae,* and/or supernatant of the microbial strain of *Lachnospiraceae* in the microbial strain, microbial agent, drug and composition provided by the present disclosure can enhance the anti-tumor activity of toxic T cells, enhance the subject's immune response, inhibit the growth of tumor cells, inhibit the spread of tumor cells, inhibit the immune escape of tumors, regulate the level of one or more symbiotic microorganisms in the subject's intestinal tract, upregulate a pro-inflammatory cytokine or chemokine, reduce the intestinal barrier permeability and/or upregulate a protective immunogenic cytokine, modulate a chemokine, and/or increase the infiltration of T cells in tumors.

The present disclosure provides use in the manufacture of a medicament for treating and/or preventing a disease mediated by HDAC activity, wherein the microbial strain of *Lachnospiraceae,* a metabolite of the microbial strain of *Lachnospiraceae,* and/or a supernatant of the microbial strain of *Lachnospiraceae* can treat or prevent the disease mediated by HDAC activity by inhibiting HDAC activity; the disease mediated by HDAC activity includes at least one of a tumor, metabolic disease, diabetes, autoimmune disease, infectious disease, central nervous disease, and inflammatory bowel disease;
preferably, the tumor includes at least one of liver cancer, colon cancer, rectal cancer, colorectal cancer, lung cancer, breast cancer, cervical cancer, ovarian cancer, pancreatic cancer, cholangiocarcinoma, kidney cancer, and fibrosarcoma;
preferably, the HDAC activity is inhibited by regulating a short-chain fatty acid/short-chain fatty acid salt; the short-chain fatty acid includes one or more of acetic acid, butyric acid, and valeric acid; more preferably, the short-chain fatty acid is butyric acid; preferably, the short-chain fatty acid salt includes one or more of acetate, propionate, and valerate; more preferably, the short-chain fatty acid salt is butyrate; preferably, the histone acetylase is class I, class II, class III and/or class IV histone acetylase.

The microbial strain, the metabolite of the microbial strain, or the supernatant of the microbial strain provided by the prevent invention prevents and/or treats at least one of a tumor, metabolic disease, diabetes, autoimmune disease, infectious disease, central nervous disease, and inflammatory bowel disease by inhibiting histone acetylase (HDAC) activity; preferably, the HDAC activity is inhibited by regulating a short-chain fatty acid/short-chain fatty acid salt; the short-chain fatty acid includes one or more of acetic acid, butyric acid, and valeric acid; more preferably, the short-chain fatty acid is butyric acid; preferably, the short-chain fatty acid salt includes one or more of acetate, propionate, and valerate; more preferably, the short-chain fatty acid salt is butyrate; preferably, the histone acetylase is class I, class II, class III and/or class IV histone acetylase.

In one aspect, the present disclosure also provides a method for preventing and/or treating a tumor, including using an effective amount of the microbial strain of *Lachnospiraceae* or metabolite of the present disclosure to treat a patient in need thereof; alternatively, using a microbial agent or drug including the strain or metabolite of the present disclosure as an active ingredient to treat a patient in need thereof. Preferably, the strain is *Lachnospiraceae* MNH 46686 strain, which was deposited with the Guangdong Microbial Culture Collection Center with a deposit name of *Lachnospiraceae sp.* MNH 46686 and a deposit number of GDMCC No: 62002.

Embodiments of the present disclosure include the following:
Embodiment 1: Use of a microbial strain of *Lachnospiraceae,* or a culture thereof, or a metabolite thereof, or a fermentation broth thereof, or a supernatant of said fermentation broth in the manufacture of a medicament for preventing and/or treating tumors, characterized in that said microbial strain of *Lachnospiraceae* belongs to a new genus and species of the family *Lachnospiraceae* (*Lachnospiraceae sp.*), said microbial strain of *Lachnospiraceae* has a 16S rDNA of the nucleotide sequence as shown in SEQ ID No. 1, or having at least 90% identity to SEQ ID No. 1, for example, at least 91%, 92%, 93%, 94%, 95%, 96%, 97%, 97.5%, 98%, 98.65%, 98.7%, or 99% identity, for example, 97% or 98.7% or 99% identity of the 16S rDNA;

The tumors include solid tumors, soft tissue tumors, hematopoietic tumors, adenomas, or metastatic tumors; preferably, the tumor is selected from at least one of liver cancer, colon cancer, rectal cancer,colorectal cancer, lung cancer, breast cancer, cervical cancer, ovarian cancer, pancreatic cancer, cholangiocarcinoma, kidney cancer, glioma, liposarcoma, melanoma, lymphoma, small cell lung cancer, squamous cell carcinoma, chondrosarcoma, and fibrosarcoma; or the tumor is a virus-induced tumor or a bacteria-induced tumor, such as a tumor caused by infection with human papillomavirus (HPV), hepatitis B or C virus (HBV, HCV), human immunodeficiency virus (HIV), Epstein-Barr virus, or a tumor caused by infection with Helicobacter pylori (HP) or Fusobacterium nucleatum.

Embodiment 2, according to the application described in embodiment 1, it is characterized in that the *Lachnospiraceae* microbial strain comprises *Lachnospiraceae* strain MNH 46686, deposited with the Guangdong Microbial Culture Collection Center, with a deposit name of *Lachnospiraceae sp.* MNH 46686 and a deposit number of GDMCC No: 62002;

Alternatively, the microbial strain of *Lachnospiraceae* has an average nucleotide identity (ANI) value of at least 95% compared to the *Lachnospiraceae* strain MNH 46686, preferably, the average nucleotide identity ANI value is 95.5%, 96%, 96.5%, 97%, 97.5%, 98%, 98.5%, 99%, 99.5%, 99.6%, 99.7%, 99.8%, 99.9% or 100%

Embodiment 3, a composition comprising a microbial strain of *Lachnospiraceae,* wherein the microbial strain of *Lachnospiraceae* belongs to a new genus and species of the family *Lachnospiraceae* (*Lachnospiraceae sp.*), said microbial strain of *Lachnospiraceae* has a 16S rDNA of the nucleotide sequence as shown in SEQ ID No. 1, or has at least 90% identity thereto, such as at least 91%, 92%, 93%, 94%, 95%, 96%, 97%, 97.5%, 98%, 98.65%, 98.7%, 99% identity, for example 97% or 98.7% or 99% identity of 16S rDNA ;
preferably, the composition is for oral administration;
preferably, the composition further comprises a pharmaceutically acceptable carrier;
preferably, the microbial strain of *Lachnospiraceae* and the pharmaceutically acceptable carrier are present in an enteric coating.

Embodiment 4, according to the composition described in embodiment 3, wherein the microbial strain of *Lachnospiraceae* comprises *Lachnospiraceae* strain MNH 46686, deposited with the Guangdong Microbial Culture Collection Center, with a deposit name of *Lachnospiraceae sp.* MNH 46686 and a deposit number of GDMCC No: 62002;

Alternatively, the microbial strain of *Lachnospiraceae* has an average nucleotide identity (ANI) value of at least 95% to the *Lachnospiraceae* strain MNH 46686, preferably, the average nucleotide identity ANI value is 95.5%, 96%, 96.5%, 97%, 97.5%, 98%, 98.5%, 99%, 99.5%, 99.6%, 99.7%, 99.8%, 99.9% or 100%.

Embodiment 5. A composition as described in any one of Embodiments 3-4, wherein it comprises live cells of the microbial strain of *Lachnospiraceae,*live bacteria, attenuated bacteria, irradiated bacteria, inactivated bacteria, a culture of said microbial strain, a metabolite of said microbial strain, a fermentation broth of said microbial strain, or a supernatant of said fermentation broth.

Embodiment 6, The composition as described in Embodiment 5, wherein it further comprises a co-drug; the co-drug from the group consisting at least one of: a chemotherapeutic agent, a photosensitizing agent, a photothermal agent, an immunotherapeutic agent, and an agent for enhancing cell therapy;
preferably, the chemotherapeutic agent includes at least one of paclitaxel, camptothecin, 5-fluorouracil, cisplatin, doxorubicin, mitomycin or epirubicin;
preferably, the photosensitizing agent includes at least one of boron dipyrrole, chlorin, or Rose Bengal;
preferably, the photothermal agent is one or more of indocyanine green, new indocyanine green, or gold nanoparticle rods;
preferably, the immunotherapeutic agent includes an Anti-PD-1 antibody, CTLA-4 antibody, PD-L1 antibody, or PD-L1 inhibitor;
more preferably, the PD-L1 inhibitor is selected from the group consisting of durvalumab, atezolizumab and avelumab;
the Anti-PD-1 antibody or PD-L1 antibody is selected from the group consisting of pembrolizumab or nivolumab;
the CTLA-4 antibody is selected from the group consisting of ipilimumab;
more preferably, the effective dose of the PD-1 inhibitor is 1~11 mg/kg; preferably, the effective dose includes: 1 mg/kg, 2 mg/kg, 3 mg/kg, 4 mg/kg, 5 mg/kg, 6 mg/kg, 7 mg/kg, 8 mg/kg or 9 mg/kg or 10 mg/kg;
preferably, the enhanced cell therapy includes CAR-T;
preferably, the content of the microbial strain in a unit dose of the composition includes 2×10⁷~9×10¹⁰ CFU/mL or 1×10⁶~2×10¹⁰ CFU/mg of the microbial strain of *Lachnospiraceae* ; preferably, the unit dose is at least one of 4×10⁷ CFU/mL, 5×10⁷ CFU/mL, 6×10⁷ CFU/mL, 7×10⁷ CFU/mL, 8×10⁷ CFU/mL, 1×10⁸ CFU/mL, 2×10⁸ CFU/mL, 3×10⁸ CFU/mL, 4×10⁸ CFU/mL, 5×10⁸ CFU/mL, 6×10⁸ CFU/mL, 7×10⁸ CFU/mL, 8×10⁸ CFU/mL, 9×10⁸ CFU/mL, 1×10⁹ CFU/mL, 2×10⁹ CFU/mL, 3×10⁹ CFU/mL, 4×10⁹ CFU/mL, 5×10⁹ CFU/mL, 6×10⁹ CFU/mL, 7×10⁹ CFU/mL, 8×10⁹ CFU/mL, 9×10⁹ CFU/mL, 2×10¹⁰ or 1.2×10⁶ CFU/mg, 4×10⁶ CFU/mg, 5×10⁷ CFU/mg, 6×10⁷ CFU/mg, 7×10⁷ CFU/mg, 8×10⁷ CFU/mg, 9×10⁷ CFU/mg, 1×10⁸ CFU/mg, 2×10⁸ CFU/mg, 3×10⁸ CFU/mg, 4×10⁸ CFU/mg , 5×10⁸ CFU/mg , 6×10⁸ CFU/mg, 7×10⁸ CFU/mg, 8×10⁸ CFU/mg, 9×10⁸ CFU/mg.

Embodiment 7. The composition according to Embodiment 5, wherein it further comprises a pharmaceutically acceptable carrier; preferably, the carrier is selected from one or more of a diluent, dispersing agent, excipient, stabilizer, lubricant, and disintegrating agent;

Preferably, the drug is in the form of a liquid preparation, a solid preparation, a capsule preparation, a sustained-release preparation, and a nano preparation.

Embodiment 8. The composition as described in Embodiment 5, wherein at least 50% of the bacteria are live, for example, at least 90% are live bacteria.

Embodiment 9. The composition as described in Embodiment 5, wherein the composition is in the form of powder, microencapsulated powder, capsule, tablet, lozenge, granule, emulsion, suspension, suppository, beverage, food, medicine or nutraceutical, food additive, dietary supplement or dairy product.

Embodiment 10. Use of the composition described in any one of Embodiments 3-9 in the manufacture of a medicament for preventing and/or treating at least one disease selected from the group consisting of metabolic diseases, diabetes, autoimmune diseases, infectious diseases, central nervous system diseases, and inflammatory bowel diseases.

Embodiment 11. Use of the composition described in any one of Embodiments 3-9 in the manufacture of a drug for anti-tumor or tumor growth inhibition , including solid tumors, soft tissue tumors, hematopoietic tumors, adenomas, or metastatic tumors;

Preferably, inhibiting tumor growth includes at least one of the following: (a) inhibiting tumor volume growth; (b) inhibiting tumor weight increase; (c) inhibiting tumor cell growth; (d) improving tumor treatment response rate; (e) improving the efficacy of immunosuppressive drug treatment, such as improving the efficacy of PD-1 drug treatment; (f) inhibiting tumor cell metastasis; (g) reducing PD-1 resistance; (h) increasing the NK ratio in peripheral blood to activate the subject's systemic immunity; (i) inhibiting HDAC activity through at least one of acetate, propionate, butyrate or valerate in SCFA; (j) inhibiting tumors through at least one of acetate, propionate, butyrate or valerate in SCFA; (k) inhibiting tumors by regulating the subject's immune system to exert an immunomodulatory effect;

The tumor includes solid tumors, soft tissue tumors, hematopoietic tumors, glandular tumors or metastatic tumors; preferably, the tumor is selected from at least one of liver cancer, colon cancer, rectal cancer, colorectal cancer, lung cancer, breast cancer, cervical cancer, ovarian cancer, pancreatic cancer, bile duct cancer, kidney cancer, glioma, liposarcoma, melanoma, lymphoma, small cell lung cancer, squamous cell carcinoma, chondrosarcoma and fibrosarcoma ; or the tumor is a tumor caused by a virus or a tumor caused by bacteria, such as human papillomavirus infection (HPV), hepatitis B or C infection (HBV, HCV), human immunodeficiency virus infection (HIV), Helicobacter pylori infection (HP), Epstein-Barr virus infection , and Helicobacter pylori (HP) infection and Fusobacterium nucleatum (F.nucleatum) infection .

Embodiment 12, Use of the composition described in any one of Embodiments 3-9 in the preparation of a medicament for treating and/or preventing a disease mediated by HDAC activity , wherein preferably, the disease mediated by HDAC activity comprises at least one of tumors, metabolic diseases, diabetes, autoimmune diseases, infectious diseases, central nervous system diseases, and inflammatory bowel disease; preferably, the tumor comprises a solid tumor, a soft tissue tumor, a hematopoietic tumor, a glandular tumor, or a metastatic tumor; preferably, the tumor is selected from liver cancer, colon cancer, rectal cancer, colorectal cancer, lung cancer, breast cancer, cervical cancer, ovarian cancer, , pancreatic cancer, bile duct cancer, renal cancer, glioma, liposarcoma, melanoma, lymphoma, small cell lung cancer, squamous cell carcinoma, chondrosarcoma and fibrosarcoma ; or the tumor is a tumor caused by viral infection or a tumor caused by bacterial infection, such as human papillomavirus infection (HPV), hepatitis B or C infection (HBV, HCV), human immunodeficiency virus infection (HIV), Epstein-Barr virus infection, and Helicobacter pylori (HP) infection , Fusobacterium nucleatum (F.nucleatum) infection caused tumors .

Embodiment 13. The microbial strain of *Lachnospiraceae* , or a culture thereof, or a metabolite thereof, or a fermentation broth thereof, or a supernatant of said fermentation broth, characterized in that said microbial strain of *Lachnospiraceae* belongs to a new genus and species of the family *Lachnospiraceae* (*Lachnospiraceae sp*.), said microbial strain of *Lachnospiraceae* has a 16S rDNA nucleotide sequence as shown in SEQ ID No. 1.

Embodiment 14, The microbial strain of *Lachnospiraceae* according to embodiment 13, wherein the microbial strain of *Lachnospiraceae* comprises *Lachnospiraceae* strain MNH 46686, deposited with the Guangdong Microbial Culture Collection Center, with a deposit name of *Lachnospiraceae sp.* MNH 46686 and a deposit number of GDMCC No: 62002.
Embodiment 15, a method for treating or preventing tumors or diabetes, comprising the step of administering to a subject in need thereof an effective amount of the *Lachnospiraceae* microbial strain described in Embodiment 13 or 14, or a culture of the *Lachnospiraceae* microbial strain, or a metabolite of the *Lachnospiraceae* microbial strain, or a fermentation broth of the *Lachnospiraceae* microbial strain, or a supernatant of the fermentation broth, or the composition described in any one of Embodiments 3-9;
Preferably, the tumor comprises a solid tumor, a soft tissue tumor, a hematopoietic tumor, a glandular tumor or a metastatic tumor; preferably, the tumor is selected from one or more of liver cancer, colon cancer, rectal cancer, colorectal cancer, lung cancer, breast cancer, cervical cancer, ovarian cancer, pancreatic cancer, bile duct cancer, kidney cancer, glioma, liposarcoma, melanoma, lymphoma, small cell lung cancer, squamous cell carcinoma, chondrosarcoma and fibrosarcoma; or the tumor is a tumor caused by a virus or a tumor caused by a bacterium, such as a tumor caused by human papillomavirus infection, hepatitis B infection or hepatitis C infection, human immunodeficiency virus infection, Helicobacter pylori infection, Epstein-Barr virus infection or Fusobacterium nucleatum infection;
Preferably, the strain prevents and / or treats tumors by inhibiting tumor growth, and the inhibition of tumor growth includes at least one of the following: ( a ) inhibiting tumor volume growth; ( b ) inhibiting tumor weight increase; ( c ) inhibiting tumor cell growth; ( d ) improving tumor treatment response rate; ( e ) improving the efficacy of immunosuppressive drug treatment, such as improving the efficacy of PD-1 drug treatment; ( f) inhibiting tumor cell metastasis; ( g ) reducing PD-1 resistance; ( h ) increasing the NK ratio in peripheral blood to activate the subject's systemic immunity; ( i ) inhibiting HDAC activity through at least one of acetic acid or acetate , propionic acid or propionate, butyric acid or butyrate, valeric acid or valeric acid in SCFA ; ( j ) inhibiting tumors through at least one short-chain fatty acid or short-chain fatty acid salt of acetic acid or acetate, propionic acid or propionate, butyric acid or butyrate, valeric acid or valeric acid in SCFA; ( k ) inhibiting tumors by regulating the subject's immune system to exert an immunomodulatory effect.

Embodiment 16. The *Lachnospiraceae* microbial strain described in embodiment 16, embodiment 13 or 14, or the culture of the *Lachnospiraceae* microbial strain, or the metabolite of the *Lachnospiraceae* microbial strain, or the fermentation broth of the *Lachnospiraceae* microbial strain, or the supernatant of the fermentation broth, or the composition described in any one of embodiments 3-9, for preventing and/or treating tumors or diabetes;
Preferably, the tumor comprises a solid tumor, a soft tissue tumor, a hematopoietic tumor, a glandular tumor or a metastatic tumor; preferably, the tumor is selected from one or more of liver cancer, colon cancer, rectal cancer, colorectal cancer, lung cancer, breast cancer, cervical cancer, ovarian cancer, pancreatic cancer, bile duct cancer, kidney cancer, glioma, liposarcoma, melanoma, lymphoma, small cell lung cancer, squamous cell carcinoma, chondrosarcoma and fibrosarcoma; or the tumor is a tumor caused by a virus or a tumor caused by a bacterium, such as a tumor caused by human papillomavirus infection, hepatitis B infection or hepatitis C infection, human immunodeficiency virus infection, Helicobacter pylori infection, Epstein-Barr virus infection or Fusobacterium nucleatum infection;
Preferably, the strain prevents and / or treats tumors by inhibiting tumor growth, and the inhibition of tumor growth includes at least one of the following: ( a ) inhibiting tumor volume growth; ( b ) inhibiting tumor weight increase; ( c ) inhibiting tumor cell growth; ( d ) improving tumor treatment response rate; ( e ) improving the efficacy of immunosuppressive drug treatment, such as improving the efficacy of PD-1 drug treatment; ( f) inhibiting tumor cell metastasis; ( g ) reducing PD-1 resistance; ( h ) increasing the NK ratio in peripheral blood to activate the subject's systemic immunity; ( i ) inhibiting HDAC activity through at least one of acetic acid or acetate , propionic acid or propionate, butyric acid or butyrate, valeric acid or valeric acid in SCFA ; ( j ) inhibiting tumors through at least one short-chain fatty acid or short-chain fatty acid salt of acetic acid or acetate, propionic acid or propionate, butyric acid or butyrate, valeric acid or valeric acid in SCFA; ( k ) inhibiting tumors by regulating the subject's immune system to exert an immunomodulatory effect.

Embodiments of the present invention also include the following:
Embodiment 1, use of a *Lachnospiraceae* microbial strain in the preparation of a drug for preventing and/or treating tumors, characterized in that the *Lachnospiraceae* microbial strain belongs to a new genus and new species (*Lachnospiraceae* sp.) of the *Lachnospiraceae* family, and the nucleotide sequence of 16S rDNA of the *Lachnospiraceae* microbial strain is shown in SEQ ID No. 1; the tumor includes at least one of liver cancer, colon cancer, rectal cancer, colorectal cancer, lung cancer, breast cancer, cervical cancer, ovarian cancer, pancreatic cancer, bile duct cancer, kidney cancer and fibrosarcoma.
Embodiment 2, according to the application described in embodiment 1, it is characterized in that the *Lachnospiraceae* microbial strain includes *Lachnospiraceae* microbial strain MNH 46686, which is preserved in Guangdong Microbiological Culture Collection Center, and the preservation name is *Lachnospiraceae* sp. MNH 46686, and the preservation number of the strain is GDMCC No: 62002.
Embodiment 3, *Lachnospiraceae* microbial strain, characterized in that the *Lachnospiraceae* microbial strain belongs to a new genus and new species (*Lachnospiraceae* sp.) of the *Lachnospiraceae* family, and the nucleotide sequence of 16S rDNA of the *Lachnospiraceae* microbial strain is shown in SEQ ID No.1.
Embodiment 4, according to the *Lachnospiraceae* microbial strain described in embodiment 3, it is characterized in that the *Lachnospiraceae* microbial strain includes *Lachnospiraceae* microbial strain MNH 46686, which is preserved in Guangdong Microbial Culture Collection Center, and the preservation name is *Lachnospiraceae* sp. MNH 46686, and the preservation number of the strain is GDMCC No: 62002.
Embodiment 5, a microbial agent, characterized in that the microbial agent comprises the *Lachnospiraceae* microbial strain described in Embodiment 3 or Embodiment 4, or a metabolite of the *Lachnospiraceae* microbial strain, or the supernatant of the *Lachnospiraceae* microbial strain.
Embodiment 6, a drug for preventing and/or treating tumors, characterized in that it comprises the *Lachnospiraceae* microbial strain described in Embodiment 3 or Embodiment 4, or a metabolite of the *Lachnospiraceae* microbial strain, or a supernatant of the *Lachnospiraceae* microbial strain;
Preferably, the unit dose of the drug comprises 9×10⁸ ~2×10⁹ CFU/mL or 1×10⁶ ~ 2×10⁹ CFU/mg of the Lachnospira microbial strain; the unit dose is preferably 9×108 CFU/mL or 1×109 CFU/mg, 9.2×10⁹ CFU/mL or 1.2×10⁹ CFU/mg, 9.3×10⁹ CFU/mL or 1.4×10⁹ CFU/mg, 9.4×10⁹ CFU/mL or 1.1×10⁹ CFU/mg, 1.2×10⁹ CFU/mL Or at least one of 1.8×10⁹ CFU/mg and 1.4×10⁹ CFU/mg or 1.6×10⁹ CFU/mg, 1.7×10⁹ CFU/mL or 1.2×10¹⁰ CFU/mg, 1.8×10⁹ CFU/mL or 1.4×10⁹ CFU/mg, 1.6×10⁹ CFU/mL or 1.6×10¹⁰ CFU/mg, 1.8×10¹⁰ CFU/mL or 1.8×10¹⁰ CFU/mg and 2×10¹⁰ CFU/mL or 2×10¹⁰ CFU/mg.
Embodiment 7. The drug according to embodiment 6, characterized in that the drug further comprises a pharmaceutically acceptable carrier; preferably, the carrier is selected from one or more of a diluent, a dispersant, an excipient, a stabilizer, a lubricant, and a disintegrant;
Preferably, the drug is in the form of a liquid preparation, a solid preparation, a capsule preparation, a sustained-release preparation, and a nano preparation.
Embodiment 8. A pharmaceutical composition, characterized in that the pharmaceutical composition comprises the microbial strain of the *Lachnospiraceae* family described in any one of Embodiments 3-4 and/or the microbial agent described in Embodiment 5 and/or the drug described in any one of Embodiments 6-7; the pharmaceutical composition also comprises a combination drug; the combination drug comprises at least one of a chemotherapeutic drug, a photosensitizer, a photothermal agent, an immunotherapy drug, and an enhanced cell therapy drug;
Preferably, the chemotherapeutic drugs include one or more of paclitaxel, camptothecin, 5-fluorouracil, cisplatin, doxorubicin, mitomycin or epirubicin;
Preferably, the photosensitizer comprises one or more of boron dipyrrole, chlorin or red bengal;
Preferably, the photothermal agent is one or more of indocyanine green, new indocyanine green or gold nanoparticle rods;
Preferably, the immunotherapy drug comprises a PD-1 antibody, a CTLA-4 antibody, a PD-L1 antibody, and a PD-L1 inhibitor; more preferably, the PD-L1 inhibitor is selected from durvalumab, atezolizumab, or avelumab; the PD-1 antibody or PD-L1 antibody is selected from pembrolizumab or nivolumab; the CTLA-4 antibody is selected from ipilimumab;
Preferably, the enhanced cell therapy comprises CAR-T;
Preferably, the unit dose of the drug comprises 9×10⁸ to 2×10⁹ CFU/mL or 1×10⁶ to 2×10⁹ CFU/mg of a *Lachnospiraceae* microbial strain; the unit dose is preferably 9×10⁸ CFU/mL or 1×10⁹ CFU/mg, 9.2×10⁹ CFU/mL or 1.2×10⁹ CFU/mg, 9.3×10⁹ CFU/mL or 1.4×10⁹ CFU/mg, 9.4×10⁹ CFU/mL or 1.1×10⁹ CFU/mg, 1.2×10⁹ CFU/mL Or at least one of 1.8×10⁹ CFU/mg and 1.4×10⁹ CFU/mg or 1.6×10⁹ CFU/mg, 1.7×10⁹ CFU/mL or 1.2×10¹⁰ CFU/mg, 1.8×10⁹ CFU/mL or 1.4×10⁹ CFU/mg, 1.6×10⁹ CFU/mL or 1.6×10¹⁰ CFU/mg, 1.8×10¹⁰ CFU/mL or 1.8×10¹⁰ CFU/mg and 2×10¹⁰ CFU/mL or 2×10¹⁰ CFU/mg.
Embodiment 9. The microbial strain as described in any one of embodiments 3-4, the microbial agent as described in embodiment 5, the medicine as described in any one of embodiments 6-7, and the pharmaceutical composition as described in embodiment 8, characterized in that the integrity of the butyrate production pathway of the *Lachnospiraceae* microbial strain is 100% and/or the strain is a high-producing butyrate and/or acetic acid strain; preferably, the microbial strain or the metabolite of the microbial strain or the supernatant of the microbial strain prevents and/or treats at least one of tumors, metabolic diseases, diabetes, autoimmune diseases, infectious diseases, central nervous system diseases, and inflammatory bowel disease by inhibiting HDAC activity; preferably, HDAC activity is inhibited by regulating short-chain fatty acids/short-chain fatty acid salts; the short-chain fatty acids include one or more of acetic acid, butyric acid, and valeric acid; more preferably, the short-chain fatty acid is butyric acid; preferably, the short-chain fatty acid salts include one or more of acetate, propionate, and valeric acid; more preferably, the short-chain fatty acid is butyrate; preferably, the histone acetylase is class I, class II, class III and/or class IV histone acetylase.

Embodiment 10. The microbial strain as described in any one of embodiments 3-4 and 9, the microbial agent as described in any one of embodiments 5 to 9, the drug as described in any one of embodiments 6-7 and 9, and the pharmaceutical composition as described in any one of embodiments 8 to 9, characterized in that the microbial bacteria of the *Lachnospiraceae* family and/or the metabolites of the microbial bacteria of the *Lachnospiraceae* family and/or the supernatant of the microbial bacteria of the *Lachnospiraceae* family can inhibit tumor growth; preferably, inhibiting tumor growth includes inhibiting tumor volume growth, inhibiting tumor weight increase, increasing the NK ratio in peripheral blood to activate mouse systemic immunity, enhancing the efficacy of immunotherapeutics and CAR-T therapeutics, and improving tumor response rate. At least one of the effects.

Embodiment 11. The microbial strain as described in any one of embodiments 3-4, 9 to 10, the microbial agent as described in any one of embodiments 5 to 10, the drug as described in any one of embodiments 6-7, 9 to 10, and the pharmaceutical composition as described in any one of embodiments 8 to 10, characterized in that the microbial bacteria of the *Lachnospiraceae* family and/or the metabolites of the microbial bacteria of the *Lachnospiraceae* family and/or the supernatant of the microbial bacteria of the *Lachnospiraceae* family can enhance the anti-tumor activity of toxic T cells, enhance the immune response of the subject, inhibit the growth of tumor cells, inhibit the spread of tumor cells, inhibit the immune escape of tumors, regulate the level of one or more symbiotic microorganisms in the intestine of the subject, upregulate proinflammatory cytokines, chemokines, reduce intestinal barrier permeability and/or upregulate protective immunogenic cytokines, regulate chemokines, and increase at least one of the infiltration effects of T cells in tumors.

Embodiment 12. Use of the microbial strain as described in any one of embodiments 3-4, 9 to 11, the microbial agent as described in any one of embodiments 5 to 11, the drug as described in any one of embodiments 6-7, 9 to 11, and the pharmaceutical composition as described in any one of embodiments 8 to 11 in the preparation of a drug for treating and/or preventing a disease mediated by HDAC activity, characterized in that the microbial bacteria of the *Lachnospiraceae* family and/or the metabolites of the microbial bacteria of the *Lachnospiraceae* family and/or the supernatant of the microbial bacteria of the *Lachnospiraceae* family inhibit HDAC activity to achieve the treatment or prevention of the disease mediated by HDAC activity; the disease mediated by HDAC activity includes at least one of tumors, metabolic diseases, diabetes, autoimmune diseases, infectious diseases, central nervous system diseases, and inflammatory bowel diseases; preferably, the tumor includes at least one of liver cancer, colon cancer, rectal cancer, colorectal cancer, lung cancer, breast cancer, cervical cancer, ovarian cancer, pancreatic cancer, bile duct cancer, kidney cancer and fibrosarcoma;

Preferably, HDAC activity is inhibited by regulating short-chain fatty acids/short-chain fatty acid salts; the short-chain fatty acids include one or more of acetate, butyrate, and valeric acid; more preferably, the short-chain fatty acid is butyrate; preferably, the short-chain fatty acid salts include one or more of acetate, propionate, and valeric acid; more preferably, the short-chain fatty acid is butyrate; preferably, the histone acetylase is class I, class II, class III and/or class IV histone acetylase.

The MNH 46686 strain herein and the MNC-686 hereinafter are equivalent to MNH 46686.

### Information on bacterial taxonomy

Use of 16s rRNA in taxonomy:
Judgment criteria: When the similarity between the 16S rRNA gene sequences of two strains is less than 97% to 98.65%, they can be judged to belong to different species.

The method for calculating the "identity":
The "identity" between the nucleic acid sequences of two nucleic acid molecules can be determined as percent identity by a known computer algorithm, such as the "FASTA" program, using default parameters, e.g., in Pearson *et al.* (Other programs include the GCG package (Devereux, J., et al., Nucleic Acids Research 12(I):387 (1984)), BLASTP, BLASTN, and FASTA, for example, the BLAST function of the NCBI database). Other commercially or publicly available programs include the DNAStar "MegAlign" program.

In some embodiments, the method further includes administering a prebiotic to the subject. In some embodiments, the prebiotic is fructooligosaccharide, galactooligosaccharide, trans-galactooligosaccharide, xylooligosaccharide, chitooligosaccharide, soy oligosaccharide, gentiooligosaccharide, isomaltooligosaccharide, mannooligosaccharide, maltooligosaccharide, mannooligosaccharide, lactulose, lactosucrose, palatinose, glycosyl sucrose, guar gum, arabic gum, tagatose, amylose, amylopectin, pectin, xylan or cyclodextrin.

The term "administration" or "administering" generally refers to the route by which a composition (e.g., a composition) is given to a subject. Examples of routes of administration include oral, rectal, topical, inhalation (nasal), or injection administration. The injection administration includes intravenous (IV), intramuscular (IM), intratumoral (IT), and subcutaneous (SC) administration. The composition or pharmaceutical composition as described herein can be administered in any form by any effective route, including, but not limited to, intratumoral, oral, parenteral, enteral, intravenous, intraperitoneal, topical, transdermal (e.g., using any standard patch), intracutaneous, intraocular, intranasal, local, non-oral, e.g. aerosol, inhalation, subcutaneous, intramuscular, buccal, sublingual, (trans)rectal, vaginal, intraarterial and intrathecal administration. The composition as described herein is administered orally, rectally, intratumorally, topically, intravesically, by injection into or near draining lymph nodes, intravenously, by inhalation or aerosol, or subcutaneously. In another preferred embodiment, the composition as described herein is administered orally, intratumorally, or intravenously. The composition as described herein is administered orally, rectally, intratumorally, topically, intravesically, by injection into or near draining lymph nodes, intravenously, by inhalation or aerosol, or subcutaneously. In another preferred embodiment, the composition as described herein is administered orally, intratumorally, or intravenously.

The "identity" between the nucleic acid sequences of two nucleic acid molecules can be determined as percent identity by a known computer algorithm, such as the "FASTA" program, using default parameters, e.g., in Pearson *et al.* (Other programs include the GCG package (Devereux, J., et al., Nucleic Acids Research 12(I):387 (1984)), BLASTP, BLASTN, and FASTA, for example, the BLAST function of the NCBI database). Other commercially or publicly available programs include the DNAStar "MegAlign" program.

The term "average nucleotide identity ANI" in the present disclosure is an indicator for comparing the genetic relationship between two genomes at the nucleotide level. ANI is defined as the average base similarity between homologous fragments of two microbial genomes.

The term "increase", "enhancement" or "improvement" refers to a change such that the difference between post-treatment and pre-treatment states is at least 10%, 20%, 30%, 40%, 50%, 60%, 70%, 80%, 90%, 2-fold, 4-fold, 10-fold, 100-fold, 10^3-fold, 10^4-fold, 10^5-fold, 10^6-fold and/or 10^7-fold as appropriate. The characteristics that may be increased include the number of immune cells, bacterial cells, stromal cells, myeloid-derived suppressor cells, fibroblasts, or metabolites; the levels of cytokines; or other physical parameters (such as tumor size).

The term "reduction", "decrease" or "decline" refers to a change such that the difference between post-treatment and pre-treatment states is at least 10%, 20%, 30%, 40%, 50%, 60%, 70%, 80%, 90%, 1/100, 1/1000, 1/10,000, 1/100,000, 1/1,000,000 or undetectable as appropriate. The characteristics that may be reduced include the number of immune cells, bacterial cells, stromal cells, myeloid-derived suppressor cells, fibroblasts, or metabolites; the levels of cytokines; or other physical parameters such as ear thickness (e.g., in animal models of DTH) or tumor size.

As used herein, the term "metabolite" refers to a compound, composition, or molecule that is used as a substrate or product in any cellular or microbial metabolic reaction, or an ion, cofactor, catalyst or nutrient from any cellular or microbial metabolic reaction.

The term "strain" refers to a member of a bacterial species that has a genetic characteristic such that it can be distinguished from closely related members of the same bacterial species. The genetic characteristic can be the absence of all or part of at least one gene, the absence of all or part of at least one regulatory region (e.g., promoter, terminator, riboswitch, or ribosome binding site), the absence ("curing" of at least one natural plasmid), the presence of at least one recombinant gene, the presence of at least one mutated gene, the presence of at least one exogenous gene (a gene from another species), at least one mutated regulatory region (e.g., promoter, terminator, riboswitch, or ribosome binding site), the presence of at least one non-natural plasmid, the presence of at least one antibiotic resistance cassette, or a combination thereof. Genetic characteristics between different strains can be identified by PCR amplification, optionally followed by DNA sequencing of the genomic region of interest or the entire genome. In cases where one strain (as compared to another strain of the same species) acquires or loses antibiotic resistance or acquires or loses biosynthesis ability (e.g., an auxotrophic strain), the strain or nutrient/metabolite can be identified by selection or counter-selection using an antibiotic.

The term "culture" in the context of the present disclosure refers to a culture obtained under anaerobic cultivation conditions using liquid culture medium; preferably, the liquid culture medium is liquid MM01 culture medium; preferably, the cultivation temperature is 37°C, the cultivation time is 24-72 hours, preferably 36-60 hours, and more preferably 48 hours.

The liquid MM01 culture medium comprises the following components:
The liquid MM01 culture medium involved in the present disclosure comprises: peptone 5 g/L, trypsinized casein 5 g/L, yeast powder 10 g/L, beef extract 5 g/L, glucose 5 g/L, K2HPO4 2 g/L, sodium acetate 2 g/L, Tween 80 1 mL/L, hemoglobin 5 mg/L, L-cysteine hydrochloride 0.5 g/L, vitamin K1 1 uL/L, and an inorganic salt solution 8 ml/L, which comprises 0.25 g of calcium chloride, 1 g of dipotassium hydrogen phosphate, 1 g of potassium dihydrogen phosphate, 0.5 g of magnesium sulfate, 10 g of sodium bicarbonate, and 2 g of sodium chloride per L of the inorganic salt solution.

The solid MM01 culture medium involved in the present disclosure comprises: peptone 5 g/L, trypsinized casein 5 g/L, yeast powder 10 g/L, beef extract 5 g/L, glucose 5 g/L, K2HPO4 2 g/L, sodium acetate 2 g/L, Tween 80 1 mL/L, hemoglobin 5 mg/L, L-cysteine hydrochloride 0.5 g/L, vitamin K1 1 uL/L, an inorganic salt solution 8 ml/L, which comprises 0.25 g of calcium chloride, 1 g of dipotassium hydrogen phosphate, 1 g of potassium dihydrogen phosphate, 0.5 g of magnesium sulfate, 10 g of sodium bicarbonate, and 2 g of sodium chloride per L of the inorganic salt solution, and agar 15 g/L.

The term "fermentation broth" in the context of the present disclosure refers to the filtered broth of bacterial strains obtained by culturing bacteria under anaerobic conditions using a liquid culture medium according to the present invention; the "supernatant" refers to a culture supernatant of the bacterial strain according to the present invention, including compounds and/or cell debris of the strain, and/or metabolites and/or molecules secreted by the strain.

The term "subject" or "patient" refers to any mammal. A subject or patient "in need thereof" as described herein refers to an individual in need of treatment (or prevention) of a disease. The mammal includes humans, laboratory animals (e.g., primate, rat, and mouse), farm animals (e.g., cow, sheep, goat, and pig), and domestic pets (e.g., dog, cat, and rodents). The subject can be a human. The subject can be a non-human mammal, including, but not limited to, dog, cat, cow, horse, pig, donkey, goat, camel, mouse, rat, guinea pig, sheep, camel, monkey, gorilla, or chimpanzee. The subject or patient can be healthy or can have a metabolic disease at any stage of development.

As used herein, the term "treating" a disease of a subject or "treating" a subject having or suspected of having a disease refers to administering a drug therapy to the subject, such as administering one or more agents, thereby reducing or preventing deterioration of at least one symptom of the disease. Therefore, in one embodiment, "treating" means inter alia delaying progression, accelerating remission, inducing remission, increasing remission, accelerating recovery, increasing the efficacy of an alternative therapy, reducing resistance to an alternative therapy, or a combination thereof.

As used herein, the term "prevention" is well-recognized in the art and, when used in connection with a condition such as local recurrence, is well known in the art. It includes an administration that reduces the occurrence or delays the onset of a symptom of a subject's medical condition relative to a subject who does not receive the composition. Therefore, prevention of cancer includes, for example, reducing the number of detectable tumors in a patient population receiving prophylactic treatment relative to an untreated control population, and/or delaying the occurrence of detectable tumors in a treated population relative to an untreated control population, for example, in a statistically and/or clinically significant amount. The composition of the present disclosure can be administered as a food product, such as a nutritional supplement. Generally, the composition of the prevent invention is used to treat humans, although they can be used to treat animals, including monogastric mammals, such as poultry, pig, cat, dog, horse or rabbit. The composition of the present disclosure can be used to enhance the growth and performance of animals. If it is administered to an animal, oral gavage can be used.

The composition or pharmaceutical composition provided by the present disclosure can include a pharmaceutically acceptable excipient, diluent, or carrier. Acceptable carriers or diluents for therapeutic use are well known in the pharmaceutical art. Examples of suitable carriers include lactose, starch, glucose, methylcellulose, magnesium stearate, mannitol, sorbitol, etc. Examples of suitable diluents include ethanol, glycerol, and water. The pharmaceutical carrier, excipient, or diluent can be selected based on the intended route of administration and standard pharmaceutical practice. The composition can include any suitable binder, lubricant, suspending agent, coating agent, solubilizer, or the like as or in addition to a carrier, excipient or diluent. Examples of suitable binders include starch, gelatin, natural sugars, and natural or synthetic gums. The natural sugars include, for example, glucose, anhydrous lactose, free-flowing lactose, β-lactose, or corn sweeteners. Natural or synthetic gums include, for example, acacia gum, tragacanth, sodium alginate, carboxymethylcellulose, or polyethylene glycol. Examples of suitable lubricants include sodium oleate, sodium stearate, magnesium stearate, sodium benzoate, sodium acetate, sodium chloride, etc. A preservative, stabilizer, dye, or even flavoring agent can be provided in the composition. Examples of preservatives include sodium benzoate, sorbic acid, or parabens. An antioxidant and suspending agent can also be used. An antioxidant and suspending agent can also be used.

In certain embodiments, the bacterial strain in the aforementioned composition provided by the present disclosure is lyophilized. In certain embodiments, the bacterial strain in the aforementioned composition provided by the present disclosure is spray dried. In certain embodiments, the bacterial strain in the aforementioned composition provided by the present disclosure is lyophilized or spray dried, and is viable. In certain embodiments, the bacterial strain in the aforementioned composition provided by the present disclosure is lyophilized or spray dried, and can partially or completely colonize the intestine. In certain embodiments, in some cases, the lyophilized bacterial strain is reconstituted prior to administration. In some cases, reconstitution is performed using a diluent as described herein.

In some embodiments, the composition provided by the present disclosure is administered orally. Oral administration may involve swallowing, whereby the compound enters the gastrointestinal tract, and/or involve buccal, lingual, or sublingual administration, whereby the compound directly enters into the bloodstream from the mouth. Pharmaceutical dosage forms suitable for oral administration include solid suppositories, solid particles, semi-solids, and liquids (including multiphasic or dispersion systems) such as tablets; soft or hard capsules containing multi- or nano-particles, liquids (e.g., aqueous solutions), emulsions or powders; lozenges (including liquid fillers); chewables; gels; rapidly dispersing dosage forms; beads; sprays; and buccal/mucoadhesive patches.

In some embodiments, the pharmaceutical formulation is an enteric formulation, i.e., a gastric juice-resistant formulation (e.g., resistant to gastric pH) suitable for delivery of the composition of the prevent invention to the intestine by oral administration. An enteric formulation may be particularly useful when the bacterium or another component of the composition is acid-sensitive, such as susceptible to degradation under gastric conditions.

In some embodiments, the enteric formulation includes an enteric coating. In some embodiments, the formulation is an enteric-coated dosage form. For example, the formulation can be an enteric-coated tablet, an enteric-coated capsule, or the like. The enteric coating can be a conventional enteric coating, such as a conventional coating for tablets, capsules, etc. used for oral delivery. The formulation can include a film coating, for example, a film layer of an enteric polymer, such as an acid-insoluble polymer. In some embodiments, the enteric formulation is enterosoluble in nature, e.g., gastrointestinally soluble, without the need for an enteric coating. In some embodiments, the formulation is an inherently enterosoluble capsule (e.g., ^{®} from Capsugel).

In some embodiments, the formulation is a soft capsule. Soft capsules are capsules that have a certain elasticity and softness due to the addition of a softener such as glycerol, sorbitol, maltitol, or polyethylene glycol present in the capsule shell. Soft capsules can be produced, for example, on the basis of gelatin or starch. Gelatin-based soft capsules are commercially available from a variety of suppliers. Soft capsules can have various shapes depending on the mode of administration, such as oral or rectal administration. For example, they can be round, oval, elliptical, or torpedo-shaped. Soft capsules can be produced by a conventional process, such as the Scherer process, Accogel process, or the dropping or blow molding process.

In certain embodiments, the composition of the present disclosure is administered to the gastrointestinal tract via a tube, such as a nasogastric tube, orogastric tube, gastric tube, jejunostomy tube (J-tube), percutaneous endoscopic gastrostomy (PEG) or a port, such as a port leading to the stomach, jejunum, or other chest wall ports suitable as access ports.

In certain embodiments of the prevent invention, the treatment according to the prevent invention is accompanied by an assessment of the patient's intestinal microbiota. If the strain of the prevent invention is not delivered and/or fails to colonize partially or completely, and thus no efficacy is observed, the treatment can be repeated. If its delivery and/or partial or complete colonization is successful and efficacy is observed, the treatment can be discontinued.

### Biological deposit information:

The MNH 46686 strain of the present disclosure has been deposited with the Guangdong Microbial Culture Collection Center with a deposit name of *Lachnospiraceae sp.* MNH 46686 and a deposit number of GDMCC No: 62002. The deposit date is November 4, 2021. The deposit address is Guangdong Institute of Microbiology, 5th Floor, No.59 Building, No. 100 Xianlie Zhong Road, Guangzhou. The proposed classification name is *Lachnospiraceae sp.* The identification by the Collection center showed that the deposited strain was viable.

### Beneficial effects:

The microbial strain of *Lachnospiraceae* provided by the present disclosure does not exist in existing reports and belongs to a new species of a new genus.

The microbial strain of *Lachnospiraceae* of the present disclosure has a complete butyric acid production pathway, and can synthesize acetic acid, isobutyric acid, butyric acid and isovaleric acid in large quantities during growth.

The microbial strain of *Lachnospiraceae* of the present disclosure can inhibit the growth of tumors (e.g., liver cancer) and can significantly reduce tumor size and weight as compared to the control group.

The strain of the present disclosure and a metabolite thereof can be used to prepare a composition for alleviating, preventing or treating a cancer (or tumor), and have very broad application prospects.

### BRIEF DESCRIPTION OF THE DRAWINGS

In order to more clearly explain the specific embodiments of the present disclosure or the technical solutions in the prior art, the accompanying drawings that need to be used in the description of the specific embodiments or the prior art will be briefly introduced below. Obviously, the drawings in the following description relate to some embodiments of the present disclosure. For those of ordinary skill in the art, other drawings can be obtained based on these drawings without exerting any creative effort.
FIG. 1 is a photograph showing the colonial morphology of the MNH 46686 strain of the present disclosure cultured on an anaerobic blood plate for 24 h.
FIG. 2 is a micrograph showing the morphology of the MNH 46686 strain of the present disclosure.
FIG. 3 is a micrograph showing the morphology of the MNH 46686 strain of the present disclosure.
FIG. 4 is a micrograph showing the Gram staining of the MNH 46686 strain of the present disclosure.
FIG. 5 is a micrograph showing the spore Gram staining of the MNH 46686 strain of the present disclosure.
FIG. 6 shows the results of resistance of the MNH 46686 strain of the present disclosure to different pH values.
FIG. 7 shows the results of resistance of the MNH 46686 strain of the present disclosure to different concentrations of NaCl.
FIG. 8 shows the results of resistance of the MNH 46686 strain of the present disclosure to different bile salts.
FIG. 9 shows the results of biochemical identification of the MNH 46686 strain of the present disclosure by API 20A.
FIG. 10 shows a phylogenetic tree constructed by comparing the strain of the present disclosure with related strains of the family *Lachnospiraceae .*
FIG. 11 shows a regulation of the immune activity of macrophages by the MNH 46686 strain.
FIG. 12 shows a regulation of the immune activity of primary PBMCs by the MNH 46686 strain.
FIG. 13A shows that the IFNβ reporter system constructed in this study has a good response to the positive drug MSA-2. FIG. 13B shows that MNH 46686 can significantly promote the transcriptional activity of IFNβ.
FIG. 14 shows a comparison of tumor tissue sizes after dissection in an animal experiment of liver cancer prevention.
FIGS. 15A to 15D show verification of the therapeutic effect of MNH 46686 on lung cancer.
FIGS. 16A to 16J show the proportions of immune cells in mouse tissues.
FIGS. 17A to 17G show correlation analyses between immune cells in mouse tissues and tumor volume at the endpoint.
FIGS. 18A to 18D show correlation analyses between immune cells in mouse tissues and tumor weight.
FIG. 19 is a graph of mouse tumor volume in an experiment of MNH 46686 in combination with Anti-PD-1.
FIG. 20 shows a comparison of mouse tumor volumes at the experimental endpoint in an experiment of MNH 46686 in combination with Anti-PD-1.
FIG. 21 shows a comparison of mouse tumor weights at the experimental endpoint in an experiment of MNH 46686 in combination with Anti-PD-1.
FIGS. 22A to 22C show graphs of mouse response rates at the experimental endpoint in an experiment of MNH 46686 in combination with Anti-PD-1.
FIG. 23 shows the inhibition of histone deacetylase (HDAC) by the MNH 46686 strain.
FIGS. 24A to 24D show the proportions of immune cells in mouse tissues. The data were analyzed by CyExpert and statistically processed by GraphPad Prism V9. Statistical analysis was performed using the T test (Student's t-test), * p < 0.05, ** p < 0.01, *** p < 0.001.
FIGS. 25A to 25D show correlation analyses between immune cells in mouse tissues and tumor weight. The data were analyzed by CyExpert and statistically processed by GraphPad Prism V9. Statistical analysis was performed using Correlation Analysis, * p < 0.05, ** p < 0.01, *** p < 0.001.
FIG. 26 is a graph of mouse tumor volume. The data in the figure are shown as mean ± SD. Statistical analysis was performed using 2-way ANOVA with Dunnett's multiple comparisons. The significance of difference is represented by *, * p < 0.05.
FIG. 27 shows a comparison of mouse tumor volumes at the experimental endpoint. The data in the figure are shown as mean ± SD. Statistical analysis was performed using one-way ANOVA with Dunnett's multiple comparisons test. The significance of difference is represented by *, * p < 0.05, ** p < 0.01, and no significance is not marked.
FIG. 28 shows a comparison of mouse tumor weights at the experimental endpoint. The data in the figure are shown as mean ± SD. Statistical analysis was performed using one-way ANOVA with Dunnett's multiple comparisons test. The significance of difference is represented by *, * p < 0.05, ** p < 0.01, and no significance is not marked.

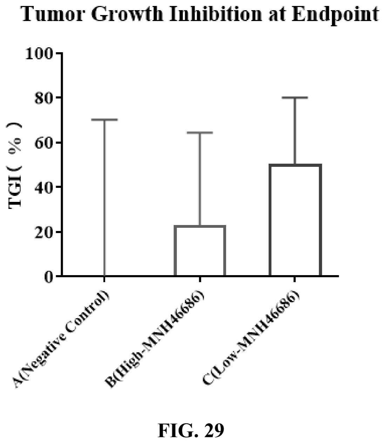

FIG. 29 shows a comparison of mouse tumor growth inhibition rates at the experimental endpoint. The data in the figure are shown as mean ± SD. Statistical analysis was performed using one-way ANOVA with Dunnett's multiple comparisons test. The significance of difference is represented by *, * p < 0.05, ** p < 0.01.
FIGS. 30A to 30C show graphs of the mouse response rate at the experimental endpoint. The data in the graphs are shown as the tumor volume (mm³) of a single mouse.

### DETAILED DESCRIPTION OF THE INVENTION

The technical solutions of the present disclosure will be clearly and completely described below with reference to the Examples. Obviously, the described Examples are part of the Examples of the present disclosure rather than all the Examples. All other Examples obtained by those of ordinary skill in the art based on the Examples of the present disclosure without exerting any creative effort fall within the scope of protection of the present disclosure.

### Example 1

### 1.1 Isolation of MNH 46686 strain

The intestinal strain *Lachnospiraceae sp.* MNH 46686 screened in the present disclosure was isolated from a stool sample of a healthy female volunteer in Guangzhou City, Guangdong Province.

Specifically, the strain was isolated as follows:
2-5 g of fresh stool was collected by the donor herself and placed into a sample collection and storage tube. After being homogenized by shaking, the processed stool sample was placed in an ice box and sent to the laboratory within 24 hours for strain isolation.

Physiological saline was dispensed in a biosafety cabinet, 9 mL/tube. Anaerobic blood agar plates (Guangzhou Huankai Bio-Technology Co.,Ltd.) with strain isolation medium were prepared and transferred to an anaerobic workstation 24 hours in advance. The sample information, type of culture medium, isolation date, etc., were marked.

The fresh stool sample was placed in the anaerobic operation station (Don Whitley Scientific H35) and mixed well by shaking on a vortex shaker for 1 min. 1 mL of the sample was pipetted into 9 mL of physiological saline, mixed well to afford a 10⁻¹ diluted solution, and then gradient diluted to 10⁻⁶ dilution for later use.

The 10⁻⁶ diluted solution was added dropwise to the isolation medium (anaerobic blood agar plate, Columbia blood agar plate and chocolate agar plate) in an amount of 100 µL/dish, and spread evenly. After the plate surface was dried, the plate was inverted and incubated at 37 °C for 3-5 days.

### 1.2 Purification of the strain

The growth status of the strain in the isolation medium was observed, and a single colony was picked up with a sterilized toothpick to purify the strain. The purified strain was anaerobically cultured at 37 °C.

The pure culture strain was prepared into 20% glycerol/water-bacterial solution and stored at -86 °C.

### Example 2. Morphological, physiological, and biochemical characteristics of MNH 46686 strain

### 2.1 Morphological characteristics of MNH 46686 strain

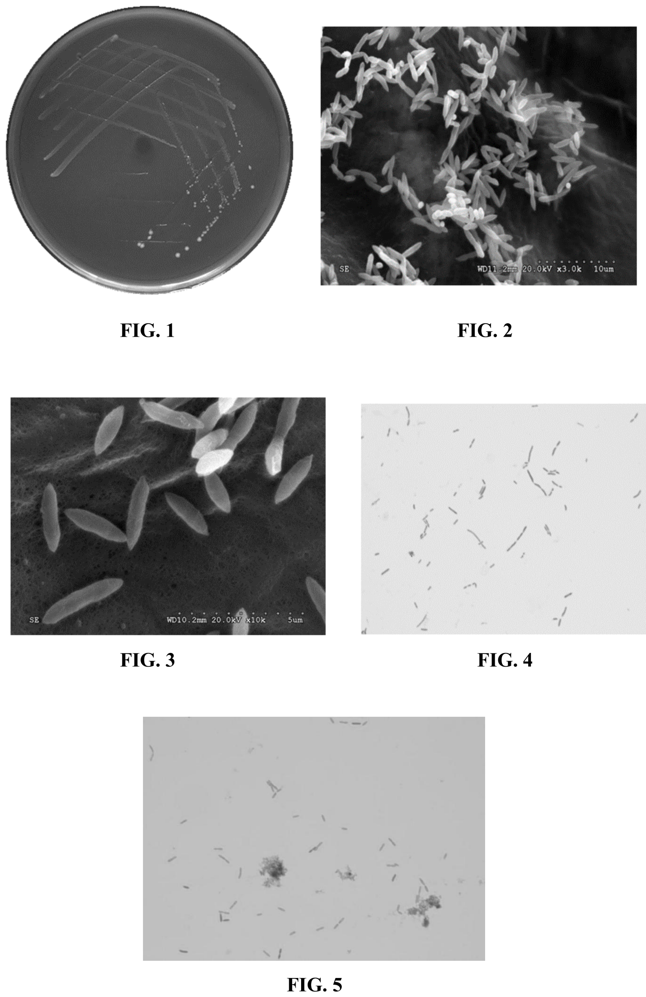

After the MNH 46686 strain was inoculated onto an anaerobic blood plate culture medium and anaerobically cultured at 37 °C for 48 h, visible colonies were formed on the anaerobic blood plate culture medium. The colonies were round, with regular and smooth edges, about 0.5 mm in diameter, pale yellow, and opaque. No secretion was formed around the colonies. The strain was Gram-negative. Morphological observation under the microscope showed that it had no flagella, was immobile, was rod-shaped, and had a length of about 2-5 µm × 20-50 µm. A photograph of the colonial morphology of the MNH 46686 strain cultured on the anaerobic blood plate for 48 h is shown in FIG. 1. Micrographs of the morphology of the MNH 46686 strain are shown in FIG. 2 (whole) and FIG. 3 (part).

A micrograph of Gram staining of the MNH 46686 strain is shown in FIG. 4, and a micrograph of spore Gram staining of the MNH 46686 strain is shown in FIG. 5 (FIGS. 4 and 5 were gray processed, and the original staining pictures were in red). The strain can produce spores and is Gram-negative.

### 2.2 Physiological and biochemical characteristics of MNH 46686 strain

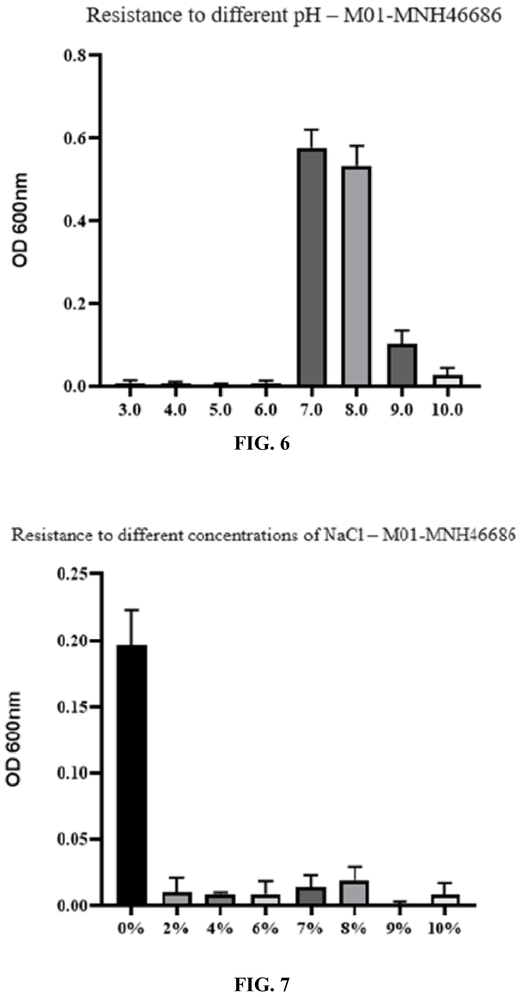

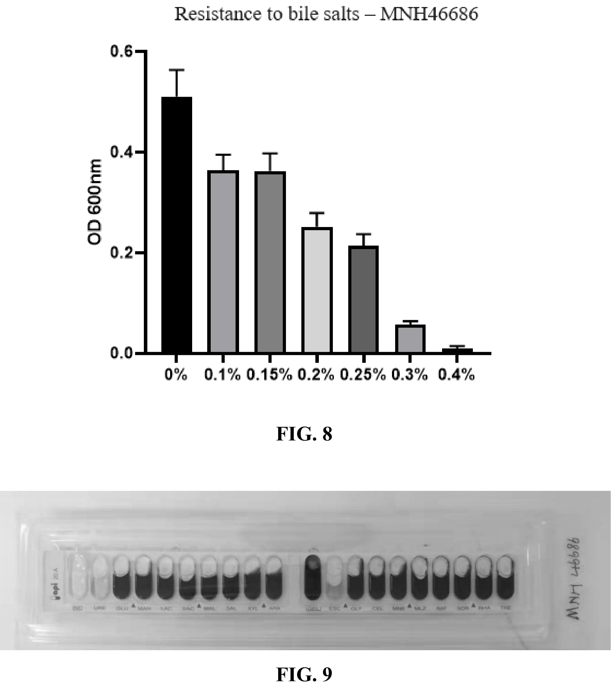

The MNH 46686 strain has a growth temperature range of 30 to 42 °C, and the optimal growth temperature is 37 °C. It can grow in the range of pH 7.0 to 9.0, and the optimal growth pH is 7.0 (the results of resistance of the strain to different pH values are shown in FIG. 6). It cannot grow on a culture media containing more than 2% NaCl (the results of resistance of the strain to different concentrations of NaCl are shown in FIG. 7). The MNH 46686 strain can survive and grow in a bile salt concentration ranging from 0% to 0.3%, but cannot grow when the bile salt concentration is greater than or equal to 0.4% (the results of resistance of the strain to different concentrations of bile salts are shown in FIG. 8).

### 2.3 Results of biochemical identification of MNH 46686 strain by API 20A

According to the instructions, API 20A reagent strip (BioMérieux) was used for testing.API 20A was used in this study. For specific experimental procedures, please see the conventional API reagent operating instructions. Culture conditions: 37 °C, anaerobically. The experimental results are shown in Table 1 and FIG. 9.

**Table 1. Results from API 20A test of MNH 46686 strain**

| Test item | Test result | Test item | Test result |
|---|---|---|---|
| IND | Negative | ESC | Negative |
| URE | Negative | GLY | No acid was produced. |
| GLU | No acid was produced. | CEL | No acid was produced. |
| MAN | No acid was produced. | MNE | No acid was produced. |
| LAC | No acid was produced. | MLZ | No acid was produced. |
| SAC | No acid was produced. | RAF | No acid was produced. |
| MAL | No acid was produced. | SOR | No acid was produced. |
| SAL | No acid was produced. | RHA | No acid was produced. |
| XYL | No acid was produced. | TRE | No acid was produced. |
| ARA | No acid was produced. | GEL | Negative |

### 2.4 Antibiotic susceptibility test of MNH 46686 strain by disk diffusion method

The results from an antibiotic susceptibility test of MNH 46686 are shown in Table 2. The MNH 46686 strain is resistant to erythromycin, ciprofloxacin, trimethoprim-sulfamethoxazole, ampicillin, ceftriaxone and lincomycin; and sensitive to antibiotics such as gentamicin, chloramphenicol, tetracycline and penicillin.

**Table 2. Results from antibiotic susceptibility test of MNH 46686 strain**

| Antibiotic | Diameter of inhibition zone (mm) | Note |
|---|---|---|
| Gentamicin (GEN) | 10.90 | Sensitiv e |
| Erythromycin (ERM) | 0.00 | Resistan t |
| Chloramphenicol (CLM) | 18.45 | Sensitiv e |
| Tetracycline (TET) | 12.01 | Sensitiv e |
| Penicillin (PEN) | 19.69 | Sensitiv e |
| Ciprofloxacin (CFX) | 0.00 | Resistan t |
| Trimethoprim-sulfamethoxazole (T/S) | 0.00 | Resistan t |
| Ampicillin (AMP) | 0.00 | Resistan t |
| Lincomycin (LIN) | 0.00 | Resistan t |
| Ceftriaxone (CTR) | 0.00 | Resistan t |

### Example 3. Identification of MNH 46686 strain

### 3.1 16S rRNA gene amplification

The genomic DNA was extracted from a fresh culture of the MNH 46686 strain. 16S rRNA gene amplification was performed using the extracted genomic DNA of the strain as a template.

The primer pair used in the 16S rRNA gene PCR of the present disclosure was as follows:
27F: 5'-AGAGTTTGATCMTGGCTCAG-3' (SEQ ID No. 2), and
1492R: 5'-TACGGYTACCTTGTTACGACTT-3' (SEQ ID No. 3).

The PCR procedure was as follows:
Pre-denaturation: 94 °C, 4 min; Denaturation: 94 °C, 50 sec; Annealing: 52 °C, 40 sec; Extension: 72 °C, 70 sec; Final extension: 72 °C, 10 min. (Note: 36 cycles).

### 3.2 16S rRNA gene sequencing

After the PCR amplification was completed, the PCR product was purified and submitted to Genewiz Co. for 16S rRNA gene sequencing to obtain the 16S rRNA gene sequence (1383 bp).

The 16S rRNA gene sequence of the MNH 46686 strain is shown as SEQ ID No. 1 :

CAGTCGAGCGAGCATTTTAAAGGAAGTTTTCGGATGGAATTTGAAATGACTG
AGCGGCGGACGGGTGAGTAACGCGTGGGTAACCTGCCTCATACAGGGGGATA
ACAGTTAGAAATGACTGCTAATACCGCATAAGCACACAGTACCGCATGGTAC

GGTGTGAAAAACTCCGGTGGTATGAGATGGACCCGCGTCTGATTAGCTGGTT
GGCGGGGTAACGGCCCACCAAGGCGACGATCAGTAGCCGACCTGAGAGGGT
GACCGGCCACATTGGGACTGAGACACGGCCCAAACTCCTACGGGAGGCAGCA
GTGGGGAATATTGCACAATGGGGGAAACCCTGATGCAGCAACGCCGCGTGAG
TGAAGAAGTATTTCGGTATGTAAAGCTCTATCAGCAGGGAAGAAAGTGACGG
TACCTGACTAAGAAGCCCCGGCTAACTACGTGCCAGCAGCCGCGGTAATACG
TAGGGGGCAAGCGTTATCCGGATTTACTGGGTGTAAAGGGAGCGTAGACGGC
ACAGCAAGTCTGAAGTGAAATCCCCGGGCTCAACCCGGGAACTGCTTTGGAA
ACTGTTGGGCTGGAGTGCTGGAGAGGCAAGCGGAATTCCTAGTGTAGCGGTG
AAATGCGTAGATATTAGGAGGAACACCAGTGGCGAAGGCGGCTTGCTGGACA
GTAACTGACGTTGAGGCTCGAAAGCGTGGGGAGCAAACAGGATTAGATACCC
TGGTAGTCCACGCCGTAAACGATGCATACTAGGTGTCGGGGAGCAAAGCTTT
TCCGTGGCCGCCCAAACGCAGTAAGTATGCCACCTGGGGAGTACGTTCGCAA
GAATGAAACTCAAAGGAATTGACGGGGACCCGCACAAGCGGTGGAGCATGT
GGTTTAATTCGAAGCAACGCGAAGAACCTTACCAAGTCTTGACATCCCCCTG
ACGAGCAAGTAACGTTGCTTTTCCTTCGGGACAGGGGAGACAGGTGGTGCAT
GGTTGTCGTCAGCTCGTGTCGTGAGATGTTGGGTTAAGTCCCGCAACGAGCGC
AACCCTTATTGTCAGTAGCCAGCAGGTAAAGCTGGGCACTCTGATGAGACTG
CCAGGGATAACCTGGAGGAAGGTGGGGATGACGTCAAATCATCATGCCCCTT
ATGATTTGGGCTACACACGTGCTACAATGGCGTAAACAAAGGGAAGCGACCC
TGCGAAGGCAAGCAAATCCCAAAAATAACGTCCCAGTTCGGATTGTAGTCTG
CAACTCGACTACATGAAGCTGGAATCGCTAGTAATCGCAGATCAGCATGCTG
CGGTGAATACGTTCCCGGGTCTTGTACACACCGCCCGTCACACCATGGGAGTC
GGAAATGCCCGAAGCCAGTGACCCAACTGCA (SEQ ID No. 1).

### 3.3 Strain identification results

The 16S rRNA gene sequence of the strain returned after sequencing was submitted to the NCBI Basic Local Alignment Search Tool for 16S rRNA gene analysis and confirmation of strain classification information.

The determined sequence and the data in GenBank were analyzed by BLAST. Comparison results showed that the strains with the highest similarity to MNH 46686 were *Lachnoclostridium pacaense* (93.77%) and *Enterocloster citroniae* (93.77%). The 16S rRNA gene sequence similarities between the MNH 46686 strain and other strains were all lower than 93.7%. The low (< 95%) 16S rRNA gene sequence similarity indicated that the MNH 46686 strain might be a new species of a new genus of the family *Lachnospiraceae .*

The 16S rRNA gene sequence of MNH 46686 was compared with those of related strains of *Lachnospiraceae* retrieved from databases such as GenBank to construct a phylogenetic tree.

Multiple sequence alignment was performed between MNH 46686 and the model strains with a relatively high 16S rRNA gene sequence similarity in the NCBI database, and then the software MEGA 5 was used to construct a phylogenetic tree (the phylogenetic tree was constructed using the maximum likelihood method). The phylogenetic tree nodes in the figure only display the Bootstrap values greater than 50%, and the superscript "T" indicates the model strains.

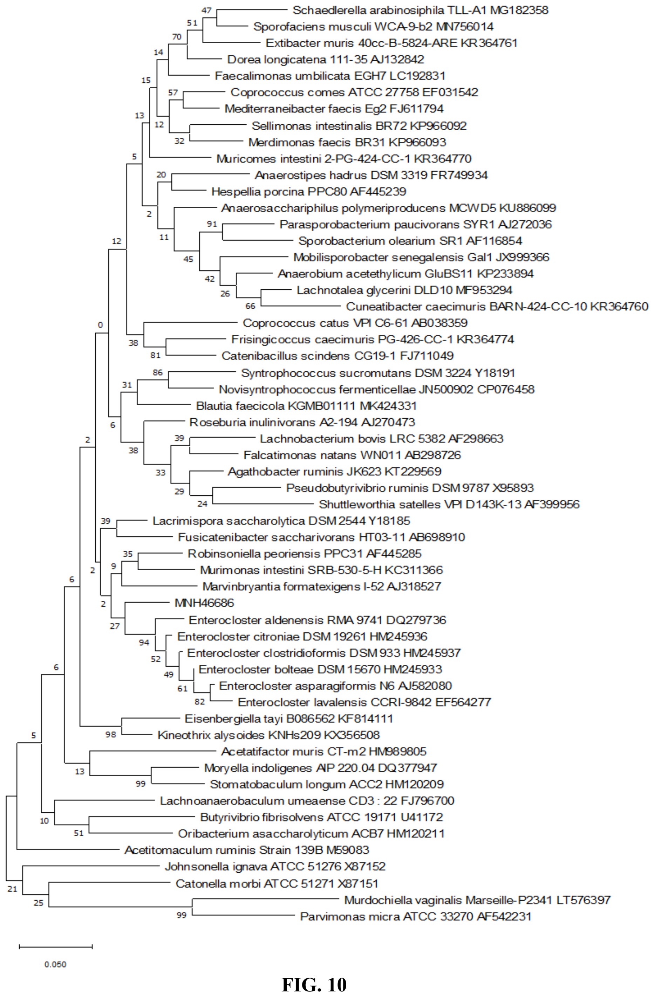

As can be seen from the phylogenetic tree (FIG. 10), the MNH 46686 strain does not cluster together with other genera of *Lachnospiraceae,* but forms a separate branch (the Bootstrap support value is 90). The phylogenetic tree analysis supports that the MNH 46686 strain is classified as a new species of a new genus of the family *Lachnospiraceae.*

### Example 4. Genomic analysis of MNH 46686 strain

The genome of the original MNH 46686 strain was fragmented by an ultrasonic method, with a fragmentation length range of ~350 bp, and then a standard DNA library construction kit (NEB UltraTM) was used to construct an Illumina sequencing library. The constructed sequencing library was subjected to paired-end 150 bp sequencing using NovaSeq (Illumina). The sequencing yielded 1.34 Gbp data, of which Q20 accounted for 97.325%.

The raw data of genome sequencing were filtered using fastp (version: 0.20.0), with the following filtration parameter: "--poly_g_min_len 10 --poly _x_min_len 10 -q 15 -u 40 -n 5 -l 50". The filtered raw data were subjected to genome assembly using SPAdes (version: v3.14.0), with the following assembly parameter "--isolate --cov-cutoff 10". The genome assembly resulted in a total gene length of 3.34 Mbp, an N50 length of 174.9 kbp, and a GC content of 51.93%.

Genomic genes were subjected to genomic gene prediction and analysis using the prokaryotic analysis software genome annotation process prokka (version: 1.14.5), with the following parameter: "--gcode 11 --evalue 1e-09". A total of 3052 CDS sequences were obtained by the prediction, with an average CDS sequence length of 977 bp.

Potential antibiotic resistance genes in the genome were analyzed using the RGI process (version: 4.2.2), in which the antibiotic resistance gene database was CARD (version: 3.0.0, https://card.mcmaster.ca/analyze/rgi). Detailed comparison information is shown in Table 3.

**Table 3. Information list of drug resistance genes**

| Gene of the strain | Resistance gene | Gene name | Identity (%) |
|---|---|---|---|
| MNC-686_01541 | ARO:3000567 | tet(40) | 99.51 |
| MNC-686_02439 | ARO:3000375 | ErmB | 98.78 |

Potential virulence factors and related genes in the genome were analyzed by using NCBI blastp (version: 2.7.1+) to compare against the virulence factor database VFDB (virulence factor database, http://www.mge.ac.cn/cgi-bin/VFs/v5/main.cgi, updated on September 19, 2019). Detailed comparison results are shown in Table 4.

**Table 4. List of potential virulence genes of MNH-46686**

| Gene of the strain | VFDB gene | Gene name | Identity (%) |
|---|---|---|---|
| MNC-686_00125 | VFG013286 | galE | 64.793 |
| MNC-686_00586 | VFG048797 | ugd | 62.408 |
| MNC-686_00593 | VFG001373 | cps41 | 73.407 |
| MNC-686_00595 | VFG001376 | cps4L | 83.793 |
| MNC-686_00597 | VFG001375 | cps4K | 66.584 |
| MNC-686_01781 | VFG000077 | clpP | 69.474 |
| MNC-686_01956 | VFG002162 | bsh | 64.375 |
| MNC-686_02546 | VFG001967 | glf | 63.26 |

Potential secondary metabolism gene clusters in the genome were analyzed using antiSMASH5 (version: 5.1.1). Detailed comparison results are shown in Table 5.

**Table 5. List of potential secondary metabolism gene clusters of MNH-46686**

| Range of gene cluster | Type | From | To | The most similar known gene cluster | Similarity |
|---|---|---|---|---|---|
| Region 7.1 | sactipeptide | 121872 | 142018 | - | - |

Potential primary metabolism gene clusters in the genome were analyzed using gutSMASH5 (version: 1.0.0). Detailed comparison results are shown in Table 6.

**Table 6. List of potential primary metabolism gene clusters of MNH-46686**

| Range of gene cluster | Type | Class | From | To | The most similar known gene cluster | Abbrev iation | Simi larit y |
|---|---|---|---|---|---|---|---|
| Region 1.1 | Rnf_complex | E-MGC | 44150 | 69270 | Rnf complex C. sporogenes | RNF | 1 |
| Region 2.1 | Glycine_reduct ase | SCFA | 159178 | 186401 | Glycine reductase C. sticklandii | GLY | 0.83 |
| Region 2.2 | TPP_AA_meta bolismTPP_fat ty_acids | Putative | 222268 | 252454 | | | |
| Region 4.1 | Pyruvate2aceta te-formate | SCFA | 21939 | 44986 | Pyruvate to acetate and formate C. acetobutylicum | PFL_ac etate | 1 |
| Region 4.2 | Putrescine2spe rmidine | Aliphati c_amine | 46656 | 69981 | Putrescine2sper midine E. rectale | SPRM | 1 |
| Region 5.1 | TPP_AA_meta bolism | Putative | 124827 | 149784 | | | |
| Region 7.1 | Others_HGD_ unassigned | Putative | 169282 | 193604 | | | |
| Region 9.1 | glutamate2buty ricsuccinate2pr opionate | SCFA | 55918 | 101483 | Glutamate to butyrate C. symbiosum | BUT | 1 |
| Region 11.1 | Fumarate2succ inate | SCFA | 16245 | 38368 | | | |
| Region 14.1 | Others_HGD_ unassigned | Putative | 7109 | 29030 | | | |
| Region 16.1 | Acetate2butyra te | SCFA | 60905 | 87005 | Acetate to butyrate C. sporogenes | BUT | 1 |

The ability of this strain to produce butyric acid was evaluated. Using the genes related to the butyric acid production pathway as described in the paper (Vital M, Howe C, Tiedje M. Revealing the Bacterial Butyrate Synthesis Pathways by Analyzing (Meta) genomic Data[J]. Mbio, 2014, 5(2): 1-11) as a reference database, the genome sequence of this strain was compared against the reference database using NCBI blastp (version: 2.7.1+). Detailed comparison results are shown in Table 7. Then, the integrity of the butyric acid production pathway was calculated. Through calculation, it was found that the integrity of the butyric acid production pathway of this strain was 100%.

**Table 7. List of potential butyric acid-producing genes of MNH-46686**

| Gene of the strain | Name of butyric acid production pathway | Gene name | Identity (%) |
|---|---|---|---|
| MNH46686_02582 | Pyruvate | Bcd | 93.75 |
| MNH46686_02578 | Pyruvate | But | 84.855 |
| MNH46686_02580 | Pyruvate | Cro | 81.783 |
| MNH46686_02584 | Pyruvate | EtfA | 89.911 |
| MNH46686_02583 | Pyruvate | EtfB | 85.769 |
| MNH46686_01795 | Glutarate | GctB | 95.539 |
| MNH46686_02581 | Pyruvate | Hbd | 92.115 |
| MNH46686_01793 | Glutarate | HgCoAd_A | 84.733 |
| MNH46686_01794 | Glutarate | HgdA | 93.718 |
| MNH46686_01807 | Glutarate | HgdB | 95.55 |
| MNH46686_02579 | Pyruvate | Thl | 86.548 |

### Example 5. Fatty acid composition analysis of MNH 46686 strain

The MNH 46686 strain was inoculated onto a TSA plate and anaerobically cultured at 37 °C for 48 h. The bacterial cells were collected and subjected to fatty acid extraction and methylation treatment. A fully automatic bacterial identification system from MIDI Co. (Microbial ID, Inc., Newark, Del) (Kroppenstedt, 1985; Meier, *et al.,* 1993) was used to perform a fatty acid composition analysis of the MNH 46686 strain.

The fatty acid composition of the experimental strain MNH 46686 is shown in the table below.

The results show that the main fatty acids (>10%) of MNH 46686 are 16-carbon saturated fatty acid (C16:0, 34.61%), 18:1 CIS 9 FAME (18.30%), 18:1c11/t9/t6 FAME (12.74%) and 18-carbon saturated fatty acid (C18:0, 10.92%). Other fatty acids and their contents are detailed in Table 8 below.

**Table 8. Fatty acid composition of MNH 46686 strain**

| RT | Respo nse | Ar/Ht | RFact | ECL | Peak Name | Perce nt | Commen t1 | Comment 2 |
|---|---|---|---|---|---|---|---|---|
| 1.627 | 9.359E +8 | 0.045 | - | 6.981 | SOLVE NT PEAK | - | < min rt | |
| 7.435 | 1271 | 0.035 | 0.987 | 13.999 | 14:0 FAME | 2.49 | ECL deviates - | Reference -0.001 |
| 10.300 | 1163 | 0.040 | 0.953 | 15.774 | 16:1 CIS 7 FAME | 2.20 | ECL deviates 0.000 | |
| 10.374 | 2856 | 0.044 | 0.952 | 15.818 | 16:1 CIS 9 FAME | 5.39 | ECL deviates 0.000 | |
| 10.678 | 18392 | 0.043 | 0.949 | 15.999 | 16:0 FAME | 34.61 | ECL deviates - 0.001 | Reference -0.002 |
| 11.507 | 1399 | 0.042 | 0.943 | 16.475 | 16:0 DMA | 2.62 | ECL deviates 0.004 | Reference 0.003 |
| 13.699 | 3448 | 0.047 | 0.931 | 17.721 | 18:2 CIS 9,12 FAME | 6.36 | ECL deviates - 0.002 | |
| 13.786 | 9918 | 0.047 | 0.931 | 17.770 | 18:1 CIS 9 FAME | 18.30 | ECL deviates - 0.001 | |
| 13.880 | 6906 | 0.048 | 0.930 | 17.824 | Sum In Feature 10 | 12.74 | ECL deviates 0.000 | 18:1c11/t9 /t6 FAME |
| 14.189 | 5929 | 0.045 | 0.929 | 17.998 | 18:0 FAME | 10.92 | ECL deviates - 0.002 | Reference -0.004 |
| 14.506 | 641 | 0.038 | 0.928 | 18.179 | Sum In Feature 11 | 1.18 | ECL deviates 0.001 | 18:2 DMA |
| 14.588 | 1133 | 0.044 | 0.927 | 18.226 | 18:1 CIS 9 DMA | 2.08 | ECL deviates 0.002 | |
| 15.014 | 614 | 0.042 | 0.926 | 18.468 | 18:0 DMA | 1.13 | ECL deviates 0.002 | |
| 16.646 | 432 | 0.038 | ---- | 19.401 | | ---- | | |
| 18.314 | 579 | 0.039 | ---- | 20.363 | | ---- | > max rt | |
| ---- | 6906 | ---- | ---- | ---- | Summed Feature 10 | 12.74 | 18:1c11/t 9/t6 FAME | UN 17.834 |
| ---- | 641 | ---- | ---- | ---- | Summed Feature 11 | 1.18 | 17:0 ISO 3OH FAME | 18:2 DMA |

### Example 6. Short-chain fatty acid (SCFA) assay of MNH 46686 strain

### 6.1 Preparation of bacterial cells

The MNH 46686 strain was inoculated into a TSB liquid culture medium and anaerobically cultured at 37 °C for 48 h. The bacterial cells were collected by centrifugation and stored at -86 °C until use.

### 6.2 Formulation of standards

Acetic acid, propionic acid, butyric acid, isobutyric acid, valeric acid, isovaleric acid, and hexanoic acid standards were weighed and formulated with ethyl acetate to eight mixed standard concentration gradients: 0.1 µg/mL, 0.5 µg/mL, 1 µg/mL, 5 µg/mL, 10 µg /mL, 20 µg/mL, 50 µg/mL and 100 µg/mL.

To 600 µL of the standard was added 25 µL of 4-methylvaleric acid at a final concentration of 500 µM as an internal standard, mixed well, and charged into an injection bottle for GC-MS detection. The injection volume was 1 µL, and the split ratio was 10: 1. Split injection was used.

### 6.3 Extraction of metabolites

The sample was thawed on ice. 80 mg of the sample was pipetted into a 2 mL glass centrifuge tube, resuspended in 900 µL of 0.5% phosphoric acid, mixed well by shaking for 2 min, and centrifuged at 14,000 g for 10 min. 800 µL of the supernatant was pipetted, to which an equal amount of ethyl acetate was added for extraction, mixed well by shaking for 2 min, and centrifuged at 14,000 g for 10 min. 600 µL of the upper organic phase was pipetted, to which 4-methylvaleric acid at a final concentration of 500 µM was added as an internal standard, mixed well, and charged into an injection bottle for GC-MS detection. The injection volume was 1 µL, and the split ratio was 10:1. Split injection was used.

### 6.4 Sample detection and analysis

The samples were separated on an Agilent DB-WAX capillary column (30 m × 0.25 mm ID × 0.25 µm) gas chromatography system. Temperature programming: the initial temperature was 90 °C, raised to 120 °C at 10 °C/min, then to 150 °C at 5 °C/min, finally to 250 °C at 25 °C/min, and maintained for 2 min. The carrier gas was helium, with a flow rate of 1.0 mL/min.

Mass spectrometry analysis was performed on an Agilent 7890A/5975C gas chromatograph-mass spectrometer. The inlet temperature was 250 °C, the ion source temperature was 230 °C, the transmission line temperature was 250°C, and the quadrupole temperature was 150 °C. An electron impact ionization (EI) source and full scan and SIM scanning modes were used, and the electron energy was 70 eV.

The chromatographic peak area and retention time were extracted using the MSD ChemStation software. A standard curve was plotted to calculate the content of short-chain fatty acids in the sample.

**Table 9. Short-chain fatty acid (SCFA) yield results of MNH 46686 strain**

| **Type of SCFA** | **Yield (µg/g)** |
|---|---|
| Acetic acid | 857.1941054 |
| Propionic acid | 14.63679879 |
| Isobutyric acid | 1711.508723 |
| Butyric acid | 1797.854335 |
| Isovaleric acid | 1415.507669 |
| Valeric acid | 0.216392053 |
| Hexanoic acid | 0.396975456 |

The test results show that the MNH 46686 strain can synthesize acetic acid, isobutyric acid, butyric acid, and isovaleric acid in large quantities during growth.

### Example 7. Regulation of the immune activity of macrophages by MNH 46686 strain

The experimental method was based on Experimental Example 1 in the prior art CN112618576B.

THP-1 cells (Wuhan Pricella Biotechnology Co., Ltd.) were treated with PMA at a final concentration of 5 ng/mL for 48 h to differentiate into M0 macrophages.

The MNH 46686 strain was added according to the ratio of MOI (viable bacterial count: cell count) = 8-10:1. Also, an M1 macrophage control group was set up (M0 macrophages were induced with 20 ng/ml IFNγ + 10 pg/ml LPS for 24 h). After culturing for 1 h, a mixture of antibiotics (Ampicillin, Streptomycin, and Colistin) was added to kill the bacteria. After culturing for an additional 23 h, the supernatant was collected.

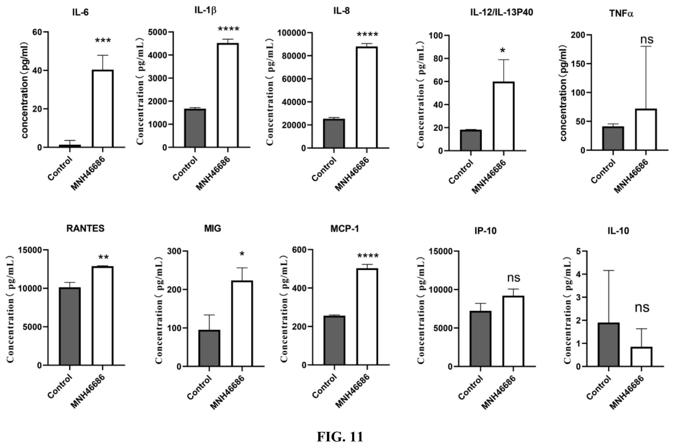

After the supernatant sample was reacted with the antibody proteins using the BD^{™} Cytometric Bead Array (CBA) Human Soluble Protein Master Buffer Kit according to the instructions, the concentrations of cytokines such as IL-10, MIG, IL-6, MCP-1, RANTES, IL-1β, IL-8 and TNFα in the supernatant were measured using a flow cytometer (see FIG. 11 for the results).

The results showed that after co-culture of M0 macrophages and MNH 46686, the macrophages clearly exhibited characteristics of differentiation into M1 macrophages. The contents of the pro-inflammatory factors IL-6, IL-1β, IL-8, and IL-12/IL-13P40 were all significantly increased, and TNFα showed an upregulation trend. The contents of the chemokines MIG, MCP-1, and RANTES were significantly increased, and IP-10 showed an upregulation trend. The anti-inflammatory factor IL-10 showed a downregulation trend.

### Example 8. Regulation of the immune activity of primary PBMCs by MNH 46686 strain

The experimental method was based on Experimental Example 2 in the prior art CN112618576B.

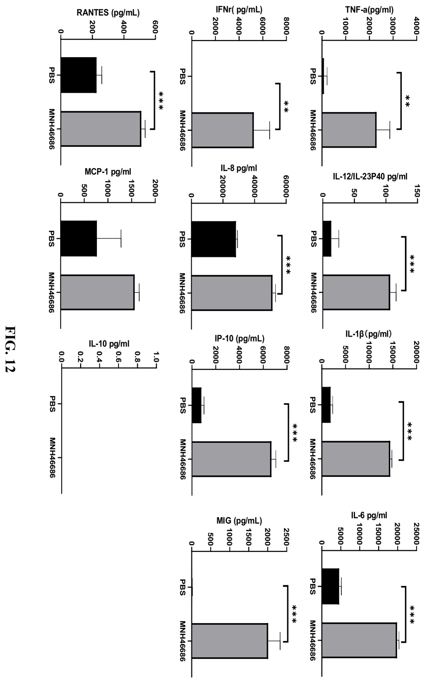

The MNH 46686 strain was co-cultured with Primary PBMC cells at a ratio of MOI = 1-5 for 4 h. A combination of antibiotics (ampicillin, streptomycin, and colistin) was added to inhibit over-proliferation of the strain. After 24 h, the supernatant was collected and the concentrations of the cytokines IFNγ, TNFα, IL-1β, IP-10 (CXCL-10), RANTES (CCL5), MIG (CXCL-9), IL- 6, MCP-1 (CCL2), IL-10, IL-12/IL-23P40, IL-8, etc. in the supernatant were measured using the BD^{™} Cytometric Bead Array (CBA) Human Soluble Protein Master Buffer Kit (see FIG. 12 for the results).

The results showed that MNH 46686 could induce a significant increase in the expression of the inflammatory factors TNFα, IL-12/IL-23P40, IL-6, IL-1β, IFNγ, IL-8, IP-10 (CXCL-10), MIG, and RANTES (CCL5). The expression of the inflammatory factor MCP-1 showed an increasing trend but did not reach a significant change. The anti-inflammatory factor IL-10 was almost not expressed.

### Example 9. Effect of MNH 46686 strain on IFNβ expression

### 1.1 Experimental methods

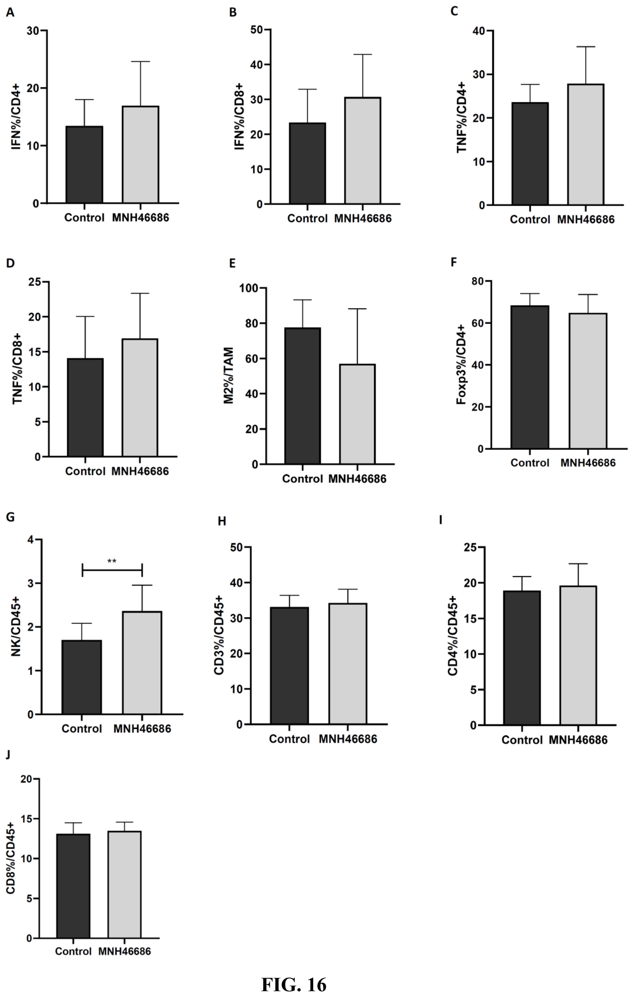

Interferon (IFN) receptor proteins are a group of cytokines secreted by host cells, which can regulate immune responses. Interferons are produced under stimulation by viruses, bacterial endotoxins, synthetic double-stranded RNAs, etc. Macrophages, lymphocytes, somatic cells, etc., in the human body can all produce interferons, in which IFNβ belongs to type I interferons and can promote the activities of NK cells, macrophages, and T lymphocytes. The experimental results in Example 12 of the present disclosure (FIGS. 16-18) showed that during the anti-tumor process of MNH 46686, NK cells were increased. Furthermore, the MNH 46686 strain of the present disclosure has been experimentally verified to be able to promote the expression of IFNβ, thereby exerting anti-viral, anti-tumor, immunomodulatory, and other effects. In order to verify whether MNH 46686 can promote the expression of IFNβ, the constructed THP-1 cells (a cell line established by Moon Co.) carrying the IFNβ-promoter reporter were used in this study to evaluate the effect of MNH 46686 on the transcriptional activity of IFNβ.

Preparation of MNH 46686 culture supernatant: The MNH 46686 strain was inoculated into MM01 liquid culture medium, anaerobically cultured at 37 °C for 48 h, and centrifuged to remove bacterial cells. The culture supernatant was filtered with a 0.22 µm filter, aliquoted, and stored at -86 °C for later use.

Positive drug group: MSA-2 (STING agonist) (from TargetMol, Cat No. T8798 (Target Molecule Corp.)) can promote the expression of IFNβ. Three gradients of 20 µM, 40 µM, and 80 µM were set.

Untreated group: DMEM complete medium (10% FBS).

High group: containing 10% by volume of MM01 bacterial culture.

MNH46686 group: containing 10% by volume of MNH 46686 bacterial culture supernatant.

The THP-1-IFNβ-promoter reporter cells were inoculated in a 96-well plate, with 1×10⁵ cells per well. The cells were treated according to the set groups. After culturing for an additional 24 h, the cells were centrifuged at 300 g for 5 min. The culture supernatant was discarded, and 50 µL of 1×luciferase detection reagent was added to allow for the reaction for 1 min. The chemiluminescence values were detected with a microplate reader. The relative luminescence values were normalized to the untreated group and compared with the relative luminescence readings of the High group to evaluate the effect of MNH 46686 on the transcriptional activity of IFNβ.

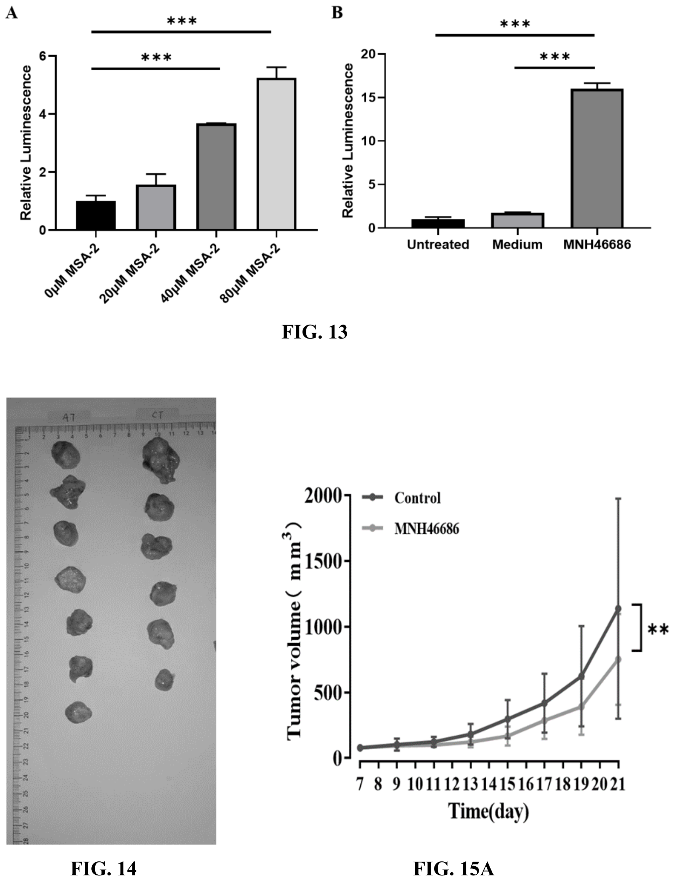

The results showed that the IFNβ reporter system constructed by the present disclosure (the reporter system mentioned in the present disclosure was obtained by inserting a reporter gene into a vector, infecting cells with the vector, and screening the cell lines expressing the reporter gene) (see Reference 1: Huashan Du, Tianmin Xu, Manhua Cui "cGAS-STING signaling in cancer immunity and immunotherapy" Biomedicine & Pharmacotherapy 133 (2021) 110972; Reference 2: Jiang, et al. "cGAS-STING, an important pathway in cancer immunotherapy" Journal of Hematology & Oncology (2020) 13:81; and Reference 3: Khiem C. Lam, et al. "Microbiota triggers STING-type I IFN-dependent monocyte reprogramming of the tumor microenvironment" Cell 184, 5338-5356) had a good response to the positive drug (FIG. 13A). This system was further used to evaluate the sample. It was found that MNH 46686 could significantly promote the transcriptional activity of IFNβ (FIG. 13B). These results indicate that MNH 46686 has potential immunomodulatory, antiviral, and antitumor effects.

### Example 10. Animal experiment on prevention of liver cancer by MNH 46686 strain

In order to verify whether the MNH 46686 strain can be used for the prevention and treatment of a tumor, a mouse syngeneic tumor model was used to conduct a liver cancer growth inhibition experiment. This study has passed the ethical review by the Laboratory Animal Care and Use Committee of Moon Biotech.

Test strain: After a glycerol cryovial of MNH 46686 strain was thawed at 37 °C, MNH 46686 was inoculated onto an anaerobic blood plate in an anaerobic workstation for activation. The activated strain was inoculated into MM01 liquid culture medium and anaerobically cultured to obtain a sufficient number of cultures. After the cultured bacterial solution was centrifuged and concentrated, the bacterial cells were resuspended in a solvent to obtain a test article whose purity and viable bacterial count (2.04×10¹⁰ CFU/mL) met the requirements of animal experiments.

Tumor cells: H22 mouse liver cancer cells, CBP69230.

Experimental animals: Experimental mice were C57BL/6J mice, 5 weeks old, 20 in total, purchased from Guangdong GemPharmatech Co., Ltd.

Animal experiments: The animals were normally fed. After the quarantine period, they were randomized into 2 groups (a control group and an experimental group) according to body weight, with 10 animals per group, and raised in separate cages. After grouping, a mouse liver cancer model was constructed by intradermal injection of H22 mouse liver cancer cells. The cell inoculation dose was 2×10⁶/mL, 0.1 mL/mouse.

On the day of completion of the inoculation (DAY 0), intragastric administration was initiated. The control group was given the culture medium, and the experimental group was given the MNH 46686 bacteria. The intragastric administration volume was 0.2 mL/mouse/time, and the administration frequency was once per day. General observation was conducted once a day during the quarantine period and after daily administration during the administration period. The animals were weighed upon arrival, at the end of the quarantine period, and three times a week during the administration period. Starting from 7 days after tumor cell inoculation (DAY 7), the tumors were measured three times a week, and the tumor growth was recorded. The endpoint of this experiment was that 19 intragastric administrations were completed. At the experimental endpoint, all mice were euthanized by cervical dislocation.

The experimental results are shown in Table 10 below.

**Table 10. Overall statistics of mice at the experimental endpoint**

| | Number of mice with successful tumor inoculation | Number of mice at experimental endpoint | Number of euthanized mice | Average weight of dissected tumors (g) | Average body weight prior to dissection (g) | Average size of dissected tumors (mm³) |
|---|---|---|---|---|---|---|
| Control group | 7 | 6 | 1 | 2.53 | 30.51 | 2372 |
| Experimental group | 10 | 7 | 3 | 1.69 | 27.55 | 1499 |

FIG. 14 shows a comparison of tumor tissue sizes after the dissection of experimental mice. The left graph in the figure shows the experimental group and the right graph shows the control group.

The tumor tissue weights after dissection of the mice were subjected to a statistical analysis. The results are shown in Table 11 below.

**Table 11. Tumor tissue weight after dissection of mice**

| **Group** | **No.** | **Tumor weight (g)** | **Mean (g)** |
|---|---|---|---|
| Control group | CT1-1 | 2.2896 | 2.52605 |
| | CT1-2 | 5.0875 | |
| | CT2-1 | 1.8192 | |
| | CT2-2 | 0.8667 | |
| | CT3-2 | 3.1895 | |
| | CT3-4 | 1.9038 | |
| Experimental group | A7-22-1 | 2.1055 | 1.69198 |
| | A7-22-3 | 1.2751 | |
| | A7-23-2 | 1.8569 | |
| | A7-23-3 | 1.2137 | |
| | A7-24-1 | 1.5365 | |
| | A7-24-2 | 2.1135 | |
| | A7-24-3 | 1.7427 | |

The results showed that the tumors in the control group grew rapidly, reaching an average weight of approximately 2.53 g on D19. The average tumor tissue weight of the experimental group was approximately 1.69 g on Day 19, and the growth rate was significantly slower than that of the control group. This experiment proves that MNH 46686 can significantly reduce the growth rate of liver cancer. It shows that the strain of the present disclosure can inhibit tumor growth and can be used for the prevention and treatment of tumors.

### Example 11. Experiment for verifying the therapeutic effect of MNH 46686 on lung cancer

### Experimental methods

In order to verify whether the MNH 46686 strain has the effect of treating a tumor, a mouse syngeneic tumor model was used to conduct a lung cancer growth inhibition experiment. This experimental protocol has passed the ethical review by the Laboratory Animal Care and Use Committee of Moon Biotech.

Test strain: After a glycerol cryovial of MNH 46686 strain was thawed at 37 °C, MNH 46686 was inoculated onto an anaerobic blood plate in an anaerobic workstation for activation. The activated strain was inoculated into an MM01 liquid culture medium and anaerobically cultured to obtain a sufficient number of cultures. After the cultured bacterial solution was centrifuged and concentrated, the bacterial cells were resuspended in a solvent to obtain a test article whose purity and viable bacterial count (1.0-2.0×10⁹ CFU/mL) met the requirements of animal experiments.

Tumor cells: LLC1 mouse lung cancer cells, CL-0140.

### (1) Verification of the therapeutic effect of MNH 46686 strain on tumors

Experimental animals: The experimental mice were C57BL/6J mice, 5 weeks old, 120 in total, purchased from Guangdong GemPharmatech Co., Ltd.

Animal experiments: The animals were normally fed. After the quarantine period, they were subcutaneously inoculated with LLC1 lung cancer cells to form an ectopic syngeneic tumor model. The cell inoculation dose was 2×10⁶/mL, 0.1 mL/animal. When the average tumor volume reached 80-100 mm³, they were randomized into 2 groups according to the tumor volume: Group A (Control) and Group F (MNH46686). Other test articles were used in Groups B-E. On the day of grouping (D1), intragastric administration was initiated. The control group was given the solvent, and group F was given MNH 46686 bacteria. The intragastric administration volume was 0.2 mL/animal/time, and the administration frequency was once per day. General observation was conducted once a day during the quarantine period and after daily administration during the administration period. The animals were weighed upon arrival, at the end of the quarantine period, and twice a week after tumor inoculation. The day of tumor cell inoculation was D1. From D5 to the day before grouping, the tumor diameter was measured once a day. After grouping, the tumor diameter was measured once every 2 days. When the average tumor volume ≥1000 mm³, the tumor diameter was measured once a day, and the tumor growth was recorded. The endpoint of this experiment was 20 days after administration. General clinical observation, weight monitoring, and tumor volume (mm³) measurements were carried out during the experiment. After the experimental endpoint was reached, all surviving mice were dissected and sampled. The tumor weight was measured, and the tumor volume was calculated. Finally, statistical analyses and comparisons were carried out for the tumor volume curve change (Tumor volume), tumor volume at the endpoint, tumor weight at the endpoint, tumor inhibition rate at the endpoint (TGI), etc. Tumor inhibition rate: (Average volume of the control group - Volume of the experimental group) / Average volume of the control group × 100%. All data are expressed in the form of mean ± SD. The GraphPad Prism 8.0.2 software was used for plotting and statistical analysis. For pairwise comparisons, a t-test (Student's t-test) was used. Multiple comparisons were used for two-way investigations, using two-way ANOVA with Sidak. The significance of difference was represented by *, * p < 0.05, ** p < 0.01, *** p < 0.001, **** p < 0.0001.

At the experimental endpoint (D21), the surviving mice were dissected, and the tumor tissues were weighed. The results are summarized in the table below.

**Table 12. Overall statistics of mice at the experimental endpoint**

| | Number of mice with successf ul tumor inoculati on | Number of mice at experimen tal endpoint | Number of euthaniz ed mice | Average weight of dissected tumors (g) | Average body weight prior to dissection (g) | Average size of dissected tumors (mm³) | TGI at experimen tal endpoint (%) |
|---|---|---|---|---|---|---|---|
| Control group | 12 | 11 | 1 | 1.33 | 23.34 | 1136.39 | 0% |
| Experi mental group | 12 | 12 | 0 | 0.74 | 23.60 | 751.78 | 36.3% |

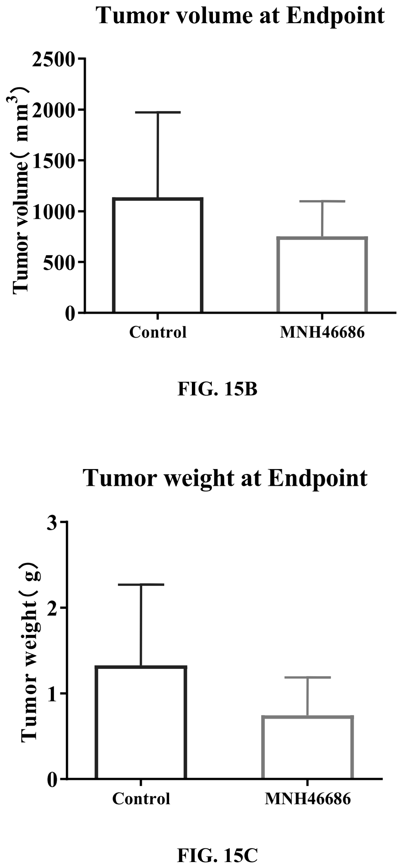

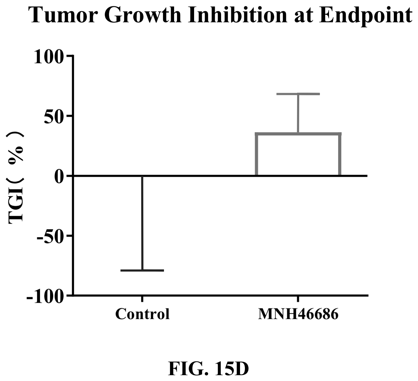

The results showed that during the experiment, the statistical analysis results of the tumor volume curve of Group F (MNH46686) were significantly smaller than those of Group A (Control) (FIG. 15A). At the experimental endpoint, the average tumor volume of the mice in the control group was 1136.39 mm³, and the average tumor tissue weight was about 1.33 g; the average tumor volume of the mice in the MNH46686 treatment group was 751.78 mm³, and the average tumor tissue weight was about 0.74 g. The tumor volume and tumor weight of the mice in the MNH46686 treatment group were significantly smaller than those of the control group (Table 12, FIGS. 15B and15C). At the experimental endpoint, the tumor growth inhibition rate (TGI) of Group F (MNO-863) was 36.3% (as shown in FIG. 15D). The tumor growth inhibition rate was significantly increased.

### Example 12. Flow cytometry results of MNH 46686

In this example, activation of the immunity of mice by MNH46686 was analyzed.

### Experimental principle:

Tissues of tumor-bearing mice were prepared into single-cell suspensions and then labeled with specific molecules with fluorescent molecular markers. The proportions of cells expressing the specific molecules can be detected in a flow cytometer. The proportional distribution of immune cells reflects the immune status of the mice to a certain extent.

### Experimental methods:

### I. Tumor tissue

### 1. Preparation of tumor single cell suspension and cell counting

0.3 g of tumor tissue was rinsed with PBS, cut into small pieces, and pulverized. The pulverized tumor tissue was placed in a 5 mL collagenase IV digestion solution system (1 mg/ml collagenase IV, 0.1 mg/ml DNase, 10% FBS) and digested at 37 °C for 30 min. The digested tissue solution was filtered through a 70 um filter membrane to prepare a mouse tumor single cell suspension, which was subjected to an on-machine test, and the total number of cells was counted.

### 2. Staining of T cells/NK cells on the surface of tumor cells

The dyes (corresponding antibody combinations) T-Live-Tumor/NK-Live-Tumor and T-Tumor/NK-Tumor were respectively added to the mouse tumor single cell suspension for staining of T cells/NK cells on the surface of tumor cells. The stained cells were resuspended in PBS and subjected to an on-machine test.

### 3. Staining of intracellular transcription factor Foxp3 in tumor cells

The dyes (corresponding antibody combinations) Treg-Live-Tumor and Treg-Surface-Tumor were successively added to the mouse tumor single cell suspension for staining. The stained cells were resuspended in PBS, then treated with a fixative and a membrane-rupturing solution successively, and stained by Treg-Foxp3-Tumor intranuclear staining according to the instructions of the detection kit. The stained cells were resuspended in PBS and subjected to an on-machine test.

### II. Peripheral blood

Peripheral blood was collected and subjected to red blood cell lysis steps: a red blood cell lysis solution was added for lysis for 5 min; PBS was added to terminate the lysis; the system was centrifuged at 500 g, and the supernatant was discarded. After the red blood cell lysis was repeated once, it was resuspended in PBS to obtain a peripheral blood single cell suspension. The dyes T-Live-Blood/NK-Live-Blood and T-Blood/NK-Blood were added to the mouse peripheral blood single cell suspension for staining of T cells/NK cells on the surface of peripheral blood cells. The stained cells were resuspended in PBS and subjected to an on-machine test.

### III. Spleen tissue

### 1. Preparation of spleen single cell suspension and cell counting

0.1 g of spleen tissue was rinsed with PBS and then subjected to tissue pulverization. The pulverized spleen tissue was resuspended in PBS and filtered through a 70 um filter membrane to prepare a mouse spleen single cell suspension, which was subjected to an on-machine test.

Red blood cell lysis solution, Spleen-Counting, pre-cooled PBS, etc. were successively added to a small amount of the mouse spleen single cell suspension as prepared above. An on-machine test was performed, and the mouse spleen single cell number was accurately counted.

Red blood cell lysis solution, PBS, etc. were successively added to the remaining mouse spleen single cell suspension as prepared above for cell processing. The processed cell suspension passed through a 300-mesh filter membrane and then was subjected to an on-machine test.

### 2. Staining of T cells/NK cells on the surface of spleen cells

The dyes T-Alexa Fluor647 anti-mouse FOXP3-SP/NK-Alexa Fluor647 anti-mouse FOXP3-SP, T-Live-SP/NK-Live-SP, and T-SP/NK-SP were respectively added to the mouse spleen single cell suspension for staining of T cells/NK cells on the surface of spleen cells. The stained cells were resuspended in PBS and subjected to an on-machine test.

### 3. Staining of intracellular transcription factor Foxp3 in spleen cells

The dyes Treg-Fcblock-SP, Treg-Live-SP, and Treg-Surface-SP were successively added to the mouse spleen single cell suspension for staining. The stained cells were resuspended in PBS and then stained using a fixative, a membrane-rupturing solution, and Treg-SP intranuclear staining successively according to the instructions of the detection kit. The stained cells were resuspended in PBS and subjected to an on-machine test.

### Experimental results:

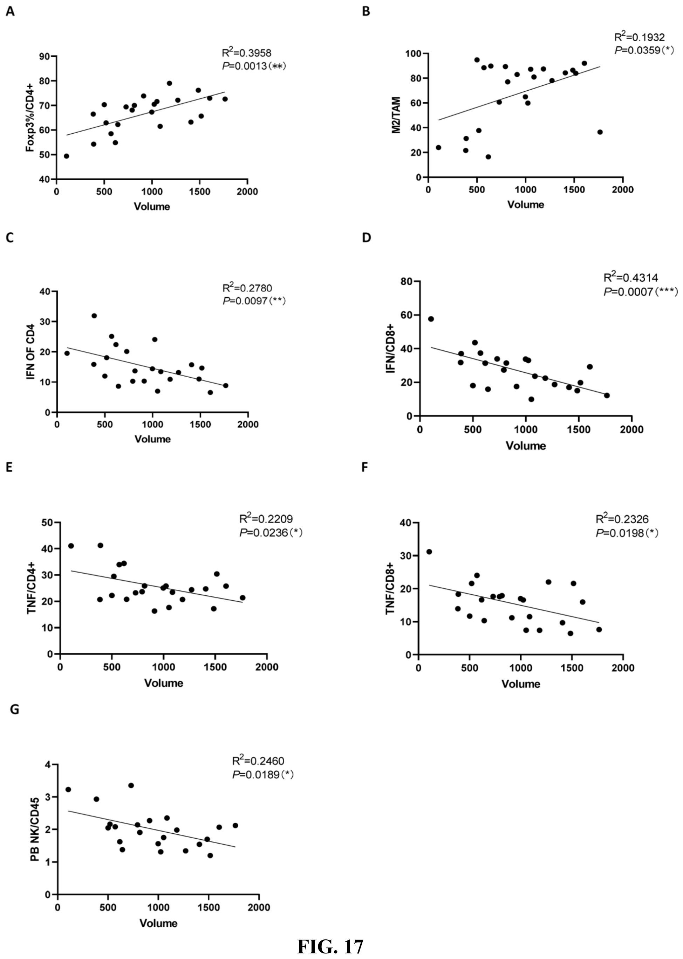

The results of the proportions of immune cells in peripheral blood are shown in FIGS. 16 and 17. As compared to the control group, the proportion of NK cells in the mouse peripheral blood was significantly upregulated, and the immune T cells such as CD45+CD3+, CD45+CD4+, and CD45+CD8+ all showed an upregulation trend (see G-J in FIG. 16). The analysis of the correlation between various immune cells in peripheral blood and tumor volume at endpoint (see FIG. 17) showed that the tumor volume at endpoint was significantly negatively correlated with the proportion of NK cells in peripheral blood, and was not significantly correlated with the proportions of other immune cells. Therefore, MNH 46686 can activate the systemic immunity of mice and exert an anti-tumor effect by increasing the proportion of NK cells in the mouse peripheral blood.

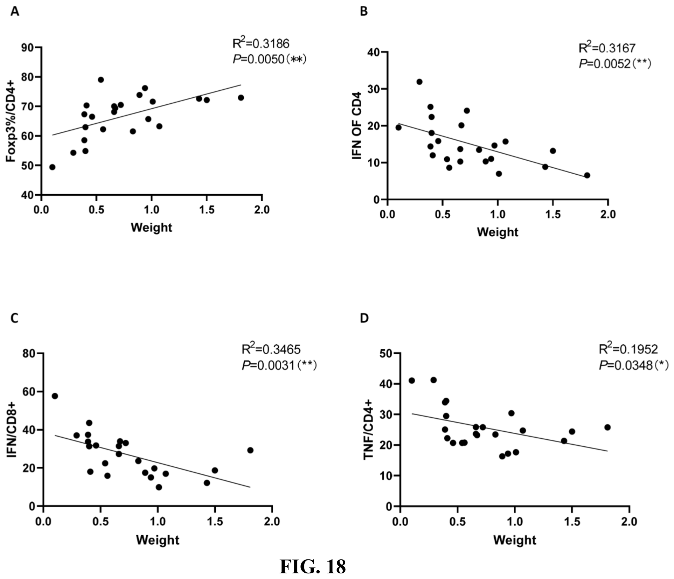

The proportions of M2 macrophages, immunosuppressive Treg cells, the killer cells CD4+IFNγ+ cells, CD8+IFNγ+ cells, CD4+TNFα+ cells, and CD8+TNFα+ cells in tumor tissue are shown in A-F in FIG. 16. As compared to the control group, the proportions of the killer cells CD4+IFNγ+ cells, CD8+IFNγ+ cells, CD4+TNFα+ cells, and CD8+TNFα+ cells in the mouse tumor tissue showed an upregulation trend, and the proportions of M2 macrophages and immunosuppressive Treg cells showed a downregulation trend. The analysis of the correlation between the above cells in the tumor tissue and the tumor volume at endpoint (see A-F in FIG. 17) showed that the tumor volume at endpoint was significantly positively correlated with the proportions of immunosuppressive Treg cells and M2 macrophages in the tumor tissue, and significantly negatively correlated with the proportions of the tumor-killing cells CD4+IFNγ+ cells, CD8+IFNγ+ cells, CD4+TNFα+ cells, and CD8+TNFα+ cells in the tumor tissue. The analysis of the correlation between immunosuppressive Treg cells, the killer cells CD4+IFNγ+ cells, CD8+IFNγ+ cells and CD4+TNFα+ cells in the tumor tissue and tumor weight (see FIG. 18) showed that the tumor weight was significantly positively correlated with the proportion of immunosuppressive Treg cells in the tumor tissue, and was significantly negatively correlated with the proportions of the tumor-killing cells CD4+IFNγ+ cells, CD8+IFNγ+ cells, CD4+TNFα+ cells, and CD8+TNFα+ cells in the tumor tissue. Therefore, MNH 46686 can activate tumor local immunity in mice and exert an anti-tumor effect by inhibiting the proportions of M2 macrophages and immunosuppressive Treg cells and increasing the proportions of the tumor-killing cells CD4+IFNγ+ cells, CD8+IFNγ+ cells, CD4+TNFα+ cells, and CD8+TNFα+ cells in mouse tumors. The MNH 46686 strain can exert an anti-tumor activity by stimulating immune system activation in mice.

### Example 13. Experiment for verifying the therapeutic effect of MNH 46686 in combination with Anti-PD-1 antibody on lung cancer

### 1. Experimental methods

In order to verify the therapeutic effect of the MNH 46686 strain in combination with an Anti-PD-1 antibody on a tumor, a mouse syngeneic tumor model was used to conduct a lung cancer growth inhibition experiment. This experimental protocol has passed the review by the Laboratory Animal Care and Use Committee of Moon Biotech.

Test strain: After a glycerol cryovial of MNH 46686 strain was thawed at 37 °C, MNH 46686 was inoculated onto an anaerobic blood plate in an anaerobic workstation for activation. The activated strain was inoculated into an MM01 liquid culture medium and anaerobically cultured to obtain a sufficient number of viable bacteria. After the cultured bacterial solution was centrifuged and concentrated, the bacterial cells were resuspended in a solvent to obtain a test article whose purity and viable bacterial count (9.23×10⁸ CFU/mL) met the requirements of animal experiments.

Tumor cells: LLC1 mouse lung cancer cells, Cat. No. CL-0140.

Experimental animals: The experimental mice were C57BL/6J mice, 5-6 weeks old, 108 in total, purchased from Guangdong GemPharmatech Co., Ltd.

Animal experiments: The animals were normally fed. After the quarantine period, they were subcutaneously inoculated with LLC1 lung cancer cells to form an ectopic syngeneic tumor model. The cell inoculation dose was 2×10⁶/mL, 0.1 mL/animal. When the average tumor volume reached 60-100 mm³, they were randomized into 8 groups according to the tumor volume: Group A (Negative Control), Group B (Positive Control, Anti-PD-1), Group E (MNH46686 & Anti-PD-1), and Groups C, D and F-H, in which other test articles were used. Administration began on the day of grouping. Group A was given the solvent and physiological saline; group B was given the solvent and the Anti-PD-1 antibody; and group E was given MNH 46686 and the Anti-PD-1 antibody. The solvent and MNH 46686 were administered intragastrically with an intragastric administration volume of 0.2 mL/animal/time and an administration frequency of once per day, for a total of 20 administrations. The Anti-PD-1 antibody and physiological saline were administered intraperitoneally with an administration dosage of 10 mg/kg and an administration frequency of once every 3 days, for a total of 6 administrations. The animals were weighed upon arrival, at the end of the quarantine period, twice a week after tumor inoculation, and on the day when intraperitoneal administration was required. The day of tumor cell inoculation was D1. From D5 to the day before grouping, the tumor diameter was measured once a day. After grouping, the tumor diameter was measured once every 3 days, and the tumor growth was recorded. The endpoint of this experiment was 23 days after administration. General clinical observation, weight monitoring, and tumor volume (mm³) measurements were carried out during the experiment. After the experimental endpoint was reached, all surviving mice were dissected and sampled. The tumor weight was measured, and the tumor volume was calculated. Finally, statistical analyses and comparisons were carried out for the tumor volume curve change (Tumor volume), tumor volume at the endpoint, tumor weight at the endpoint, tumor growth inhibition rate at the endpoint (TGI), etc. Tumor growth inhibition rate (TGI) = 1 - (Volume of individual tumor - Volume of individual tumor when grouped) / (Average tumor volume of Group A - Average tumor volume of Group A when grouped) × 100%. All data are expressed in the form of mean ± SD. The GraphPad Prism 8.0.2 software was used for plotting and statistical analysis. For mutual comparison of more than three groups, the One-Way ANOVA method with Tukey's multiple comparisons test was used for analysis. For two-way investigations, two-way ANOVA with Sidak multiple comparisons was used. The significance of difference was represented by *, * p < 0.05, ** p < 0.01, *** p < 0.001, **** p < 0.0001.

### 2. Experimental results of treatment of lung cancer with MNH 46686 strain in combination with Anti-PD-1 antibody

At the experimental endpoint (D24), the surviving mice were dissected and the tumor tissues were weighed. The results are summarized in Table 13.

**Table 13. Overall statistics of mice at the experimental endpoint**

| Group | Number of enrolled mice | Number of mice at experimenta l endpoint | Number of euthani zed mice | Average weight of dissected tumors (g) | Averag e body weight prior to dissecti on (g) | Average tumor size at endpoint (mm³) |
|---|---|---|---|---|---|---|
| Group A (Negative Control) | 10 | 10 | 0 | 1.28 | 24.81 | 1279.80 |
| Group B (Positive Control) | 10 | 10 | 0 | 0.92 | 25.43 | 710.29 |
| Group E (MNH46686 & Anti-PD-1 antibody) | 8 | 8 | 0 | 0.47 | 24.28 | 346.99 |

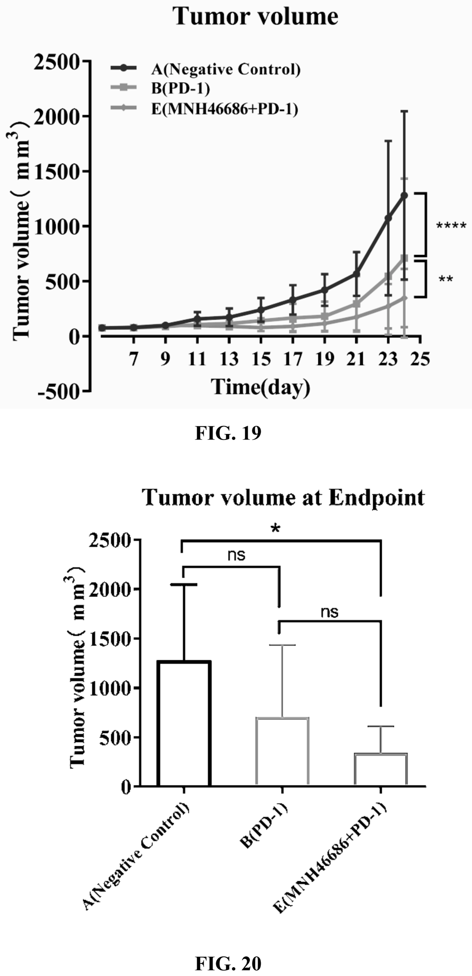

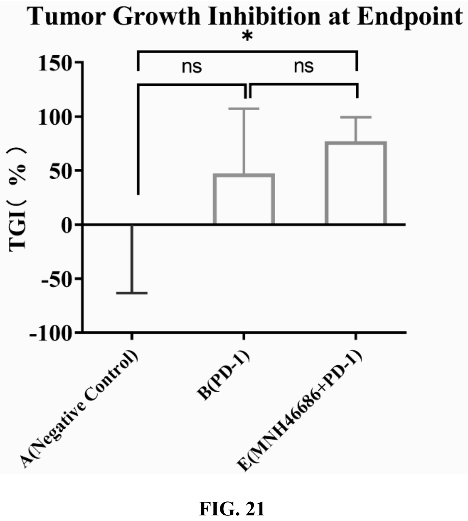

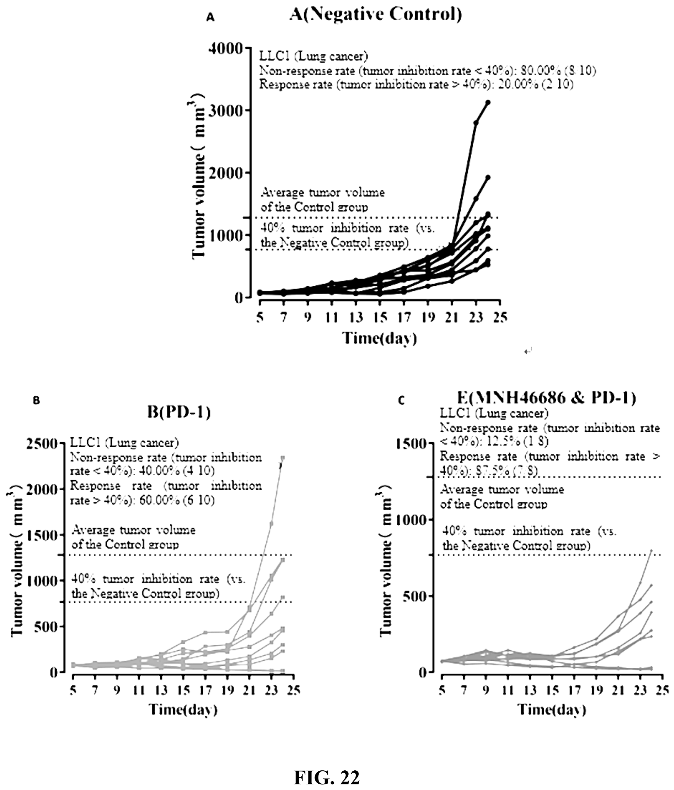

The results showed that during the experiment, the statistical analysis results of the tumor volume curve of Group E (MNH46686 & Anti-PD-1) were significantly smaller than those of Group A (Negative Control) and Group B (Positive Control) (FIG. 19). At the experimental endpoint, the average tumor volume of the mice in the negative control group was 1279.80 mm³, and the average tumor tissue weight was about 1.28 g; the average tumor volume of the mice in the positive control group (Positive Control) was 710.29 mm³, and the average tumor tissue weight was about 0.92 g; the average tumor volume of the mice in the MNH46686 & Anti-PD-1 antibody treatment group was 346.99 mm³, and the average tumor tissue weight was about 0.47 g. As compared to the negative control group, the tumor volume and tumor weight at the endpoint of the mice in the MNH46686 & Anti-PD-1 antibody treatment group were significantly downregulated. As compared to the positive control group, the tumor volume and tumor weight at the endpoint of the mice in the MNH46686 & Anti-PD-1 antibody treatment group both showed a downregulation trend (FIGS. 20 and 21). At the experimental endpoint, the tumor growth inhibition rate (TGI) of Group B (Positive Control) was 47.3%, and the tumor growth inhibition rate (TGI) of group E (MNH46686 & Anti-PD-1) was 77.2%, which was significantly higher than that of the positive control group (FIG. 22). At the experimental endpoint, the response rate of Group A (Negative Control) was 20.0%, the response rate of Group B (Positive Control) was 60.0%, and the response rate of Group E (MNH46686 & Anti-PD-1) was 87.5%. MNH 46686 in combination with an Anti-PD-1 antibody significantly improved the response rate of tumor treatment (FIG. 22). The data in the figures are shown as mean ± SD. Statistical analysis was performed using 2-way ANOVA with Sidak multiple comparisons. The significance of the difference was represented by *, * p < 0.05.

### Example 14. Inhibitory effect of MNH 46686 strain on the activity of histone deacetylases (HDAC)

In order to verify whether MNH 46686 has an inhibitory effect on the activity of histone deacetylase, the HDAC Inhibitor Drug Screening Kit (Fluorometric) purchased from Abcam Co. was used in this study to detect the inhibitory effect on HDAC activity *in vitro.*

**Preparation of MNH 46686 culture supernatant:** The MNH 46686 strain was inoculated into a liquid culture medium, anaerobically cultured at 37 °C for 48 h, and centrifuged to remove bacterial cells. The culture supernatant was filtered with a 0.22 µm filter, aliquoted, and stored at -80 °C for later use.

**Preparation of test samples:** 1) In the Control group, MM01 culture medium was diluted 10 times with PBS to obtain 10% MM01; 2) Positive control group: TSA, an HDAC inhibitor, was diluted with PBS to a final concentration of 40 µM; 3) MNH 46686 group, the MNH 46686 culture supernatant was diluted 10 times with PBS to obtain an experimental sample containing 10% bacterial supernatant.

**Preparation of reaction reagent for HDAC detection:** An appropriate amount of detection reaction system was prepared according to the instructions of the kit. 50 µL of the reaction reagent was required for each reaction.

**Detection of HDAC activity:** 50 µL of the test sample was added to a 96-well white plate, and then 50 µL of the reaction reagent was added respectively. They were mixed well and incubated at 37 °C for 30 min. 10 µL of Lysine Developer was added to the reaction well and mixed well to terminate the reaction. The plate was incubated at 37 °C for 30 min. Finally, the fluorescence intensity of the sample was detected using a microplate reader with the following microplate reader settings: Ex. = 350-380 nm, and Em. = 440-460 nm. To analyze the HDAC inhibitory activity of the sample, the fluorescence intensity value of the Control was set as 100%. The fluorescence intensities of the positive control group and the MNH 46686 group were respectively divided by that of the Control and then multiplied by 100% to obtain the relative HDAC activities.

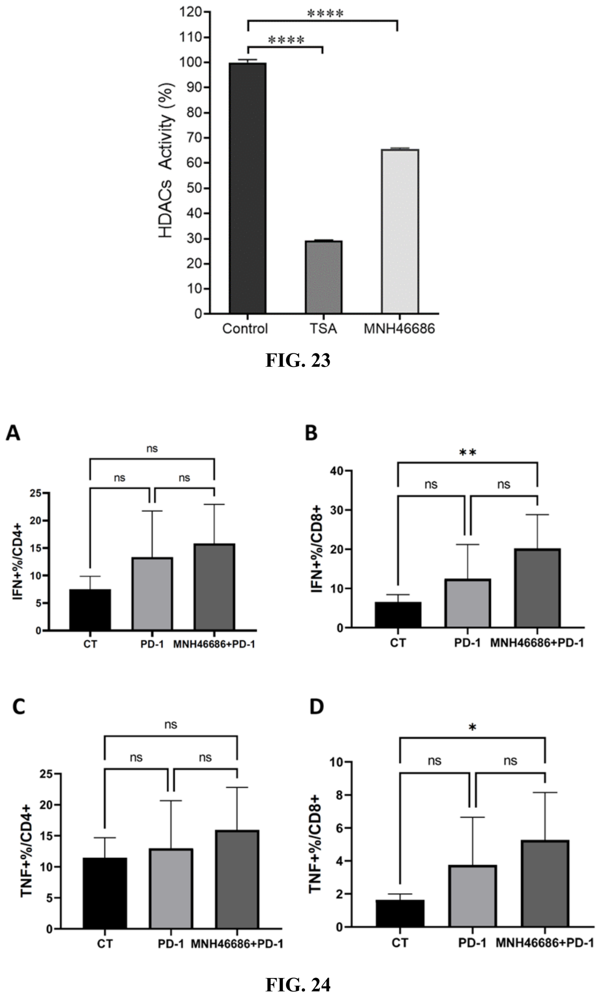

The results (see FIG. 23) showed that, as compared to the HDAC activity in the Control group, the supernatant of MNH 46686 had a significant inhibitory effect on HDAC activity, which was similar to the effect of TSA in the HDAC inhibitor control group. These results suggest that MNH 46686 can inhibit HDAC activity, and MNH 46686 can be used to prevent or treat diseases mediated by HDAC activity by inhibiting HDAC activity.

The functions of HDACs in cancers are related to the aberrant expression or function of the genes that promote cell proliferation and tumorigenic phenotypes. In some cancers, HDACs primarily regulate the onset of the cancers and have been described as oncogenes. Reducing or inhibiting the activity of HDAC expression has been shown to have multiple anti-cancer effects, such as cell cycle arrest, inhibition of proliferation, apoptosis, differentiation, senescence, and disruption of angiogenesis. Therefore, the MNH 46686 strain of the present disclosure has an HDAC inhibitory activity, suggesting that the drug or composition of the present disclosure can be used to prevent or treat tumors or cancers mediated by HDAC activity. Since the genes regulated by HDAC play an important role in angiogenesis, the drug or composition of the present disclosure can be used to prevent tumor metastasis.

Diabetes is a group of diseases in which a low level of insulin and/or peripheral insulin resistance leads to hyperglycemia. Inhibition of HDAC has been proposed to treat diabetes through multiple mechanisms, including inhibition of Pdxl (Park, et al., 2008, J Clin Invest, 118, 2316-24), and enhancement of the expression of the transcription factor Ngn3 to increase the endocrine repertoire, progenitor cells (Haumaitre, et al., 2008, Mol Cell Biol, 28, 6373-83), enhancement of insulin expression (Molsey, et al., 2003, J Biol Chem, 278, 19660-6), etc. HDAC inhibition is also a promising therapy for advanced diabetic complications such as diabetic nephropathy and retinal ischemia (Christensen, et al., 2011, Mol Med, 17(5-6), 370-390). Therefore, the composition of the present disclosure can be used to treat or prevent diabetes mediated by HDAC activity.

The composition of the present disclosure can be used to treat or prevent diabetes. In a preferred embodiment, the composition of the prevent invention can be used to treat or prevent diabetes mellitus type I. In a preferred embodiment, the composition of the prevent invention can be used to treat or prevent diabetes mellitus type II. In certain embodiments, the composition of the prevent invention can be used for the treatment or prevention of diabetes, wherein the treatment or prevention is accomplished by reducing or preventing HDAC activation.

The MNH 46686 strain of the present disclosure can inhibit HDAC activity and activate T cells, thereby achieving an anti-tumor effect.

The composition of the present disclosure has an HDAC inhibitory activity and therefore is useful for treating inflammatory bowel disease mediated by HDAC activity.

### Example 15. Analysis of the activation of mouse immunity by MNH 46686 in combination with Anti-PD-1

Tissues of tumor-bearing mice were prepared into single-cell suspensions and then labeled with specific molecules with fluorescent molecular markers. The proportions of cells expressing the specific molecules can be detected in a flow cytometer. The proportional distribution of immune cells reflects the immune status of the mice to a certain extent.

### Experimental methods:

The tumor tissue was rinsed with PBS, cut into small pieces, and pulverized. The pulverized tumor tissue was placed in a 5 mL collagenase IV digestion solution system (1 mg/ml collagenase IV, 0.1 mg/ml DNase, 10% FBS) and digested at 37 °C for 30 min. The digested tissue solution was filtered through a 70 um filter membrane to prepare a mouse tumor single cell suspension, which was subjected to an on-machine test, and the total number of cells was counted. A stimulant was added to the mouse tumor single cell suspension for stimulation for 4 h. The cells were washed and resuspended, and then the dyes (corresponding antibody combinations) Live-Tumor and IFN/TNF-Surface-Tumor were successively added for staining. The stained cells were resuspended in PBS, then treated with a fixative and a membrane-rupturing solution successively, and stained by IFN/TNF-Tumor intranuclear staining according to the instructions of the detection kit. The stained cells were resuspended in PBS and subjected to an on-machine test.

The flow cytometry data were processed by CyExpert and statistically processed by GraphPad Prism V9. Statistical analysis was performed using one-way ANOVA with Dunnet's multiple comparisons. ns: not significant, * p < 0.05, ** p < 0.01, *** p < 0.001.

### Experimental results:

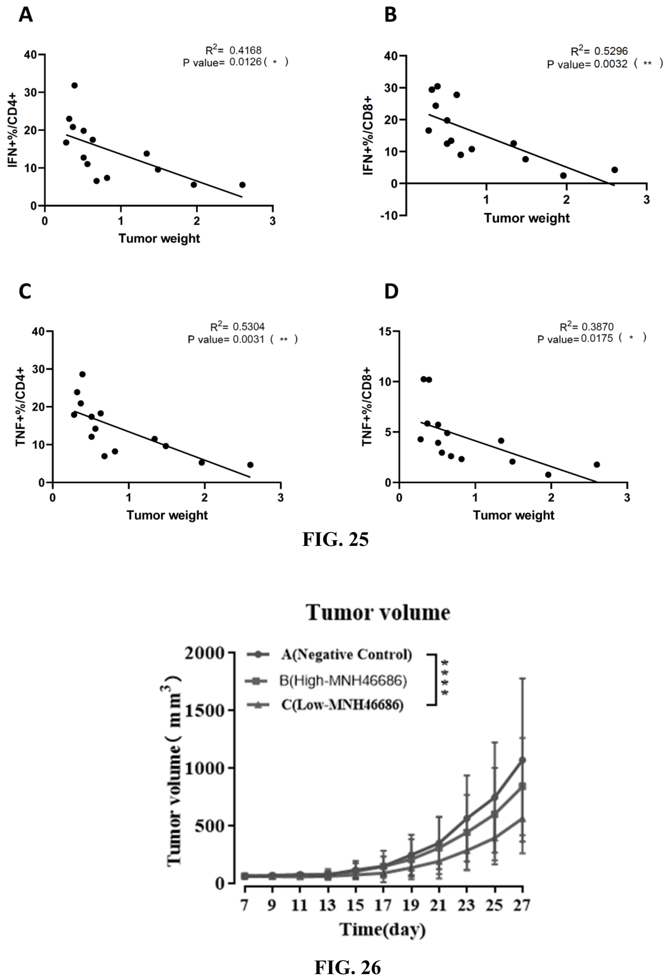

For the immune cells CD4+IFNγ+ cells, CD8+IFNγ+ cells, CD4+TNFα+ cells, and CD8+TNFα+ cells with a tumor killing effect in tumor tissue (see FIGS. 24A-24D), as compared to the positive control group (the Anti-PD-1 group), the proportions of the killer cells CD4+IFNγ+ cells, CD8+IFNγ+ cells, CD4+TNFα+ cells, and CD8+ TNFα+ cells in tumor tissues of the mice in the MNH46686 + Anti-PD-1 group showed an upregulation trend. The analysis of the correlation between the above cells in tumor tissues and tumor weight at endpoint of the positive control group (the Anti-PD-1 group) and the MNH46686 + Anti-PD-1 group (see FIGS. 25A-25D) showed that the tumor weight was significantly negatively correlated with the proportions of the tumor-killing cells CD4+IFNγ+, CD8+IFNγ+ cells, CD4+TNFα+ cells, and CD8+TNFα+ cells in the tumor tissue. Therefore, MNH 46686 can activate tumor local immunity in mice and enhance the anti-tumor effect of the Anti-PD-1 antibody by increasing the tumor-killing cells CD4+IFNγ+ cells, CD8+IFNγ+ cells, CD4+TNFα+ cells, and CD8+TNFα+ cells.

### Example 16. Exploratory experiment on the dose-effect relationship of MNH 46686 strain in lung cancer

### 1.2.1 Experimental methods

In order to explore the dose-effect relationship of the MNH 46686 strain in tumor treatment, a mouse syngeneic tumor model was used to conduct a lung cancer growth inhibition experiment. This experimental protocol has passed the review by the Laboratory Animal Care and Use Committee of Moon Biotech.

Test strain: After a glycerol cryovial of MNH 46686 strain was thawed at 37 °C, MNH 46686 was inoculated onto an anaerobic blood plate in an anaerobic workstation for activation. The activated strain was inoculated into an MM01 liquid culture medium and anaerobically cultured to obtain a sufficient number of viable bacteria. After the cultured bacterial solution was centrifuged and concentrated, the bacterial cells were resuspended in a solvent to obtain a test article whose purity and viable bacterial count (4.1×10¹⁰ CFU/mL) met the requirements of animal experiments.

Tumor cells: LLC1 mouse lung cancer cells, Cat. No. CL-0140.

Experimental animals: The experimental mice were C57BL/6J mice, 5-6 weeks old, 60 in total, purchased from Guangdong GemPharmatech Co., Ltd.

Animal experiments: The animals were normally fed. After the quarantine period, they were subcutaneously inoculated with LLC1 lung cancer cells to form an ectopic syngeneic tumor model. The cell inoculation dose was 2×10⁶/mL, 0.1 mL/animal. When the average tumor volume reached 60-100 mm³, they were randomized into 3 groups according to the tumor volume: Group A (Negative Control), Group B (High - MNH46686), and Group C (Low - MNH46686). Administration began on the day of grouping. The negative control group A (Negative Control) was given the negative control PBS-Cys, Group B (high - MNH46686) was given a 10⁻¹ diluted solution of MNH 46686 (8.2×10⁸ CFU/animal), and Group C (Low - MNH46686) was given a 10⁻² diluted solution of MNH 46686 (8.2×10⁷ CFU/animal) by intragastric administration, with an intragastric administration volume of 0.2 mL/animal/time and an administration frequency of once per day, for a total of 21 administrations. General clinical observation was conducted once a day during the experiment. The animals were weighed upon arrival, at the end of the quarantine period, and twice a week after tumor inoculation. From D5 to the day before grouping, the tumor diameter was measured once a day. After grouping, the tumor diameter was measured once every 2 days. When the average tumor volume of any group reached or exceeded 1000 mm³, and the average tumor volume of any one of Group B and Group C was significantly smaller than that of Group A or the average tumor growth inhibition rate (TGI) of this group reached or exceeded 30%, the experiment could be terminated as appropriate. Alternatively, if the average tumor volume of any group of mice reached or exceeded 2000 mm³, then the experiment should be terminated. After the experimental endpoint was reached, all surviving mice were dissected and sampled or euthanized. The tumor weight was measured, and the tumor volume was calculated. Finally, statistical analyses and comparisons were carried out for the tumor volume curve change (Tumor volume), tumor volume at the endpoint, tumor weight at the endpoint, tumor growth inhibition rate at the endpoint (TGI), etc. Tumor growth inhibition rate (TGI) = 1 - (Volume of individual tumor - Volume of individual tumor when grouped) / (Average tumor volume of Group A - Average tumor volume of Group A when grouped) × 100%. All data are expressed in the form of mean ± SD. The GraphPad Prism 8.0.2 software was used for plotting and statistical analysis. For mutual comparison of more than three groups, the One-Way ANOVA method with Dunnett's multiple comparisons test was used for analysis. Multiple comparisons were used for two-way investigations, using two-way ANOVA with Sidak. The significance of difference was represented by *, * p < 0.05, ** p < 0.01, *** p < 0.001, **** p < 0.0001.

### 1.2.2 Experimental results of dose-effect exploration of MNH 46686 strain in lung cancer

At the experimental endpoint (D27), the surviving mice were dissected, and the tumor tissues were weighed. The results are summarized in Table 14.

**Table 14. Overall statistics of mice at the experimental endpoint**

| Group | Number of enrolled mice | Number of mice at experimenta 1 endpoint | Number of euthanized mice | Average weight of dissecte d tumors (g) | Average body weight prior to dissection (g) | Average tumor size at endpoint (mm³) |
|---|---|---|---|---|---|---|
| Group A (Negative Control) | 10 | 9 | 1 | 1.39 | 23.97 | 1067.54 |
| Group B (High - MNH46686) | 10 | 9 | 1 | 1.18 | 23.71 | 838.01 |
| Group C (Low - MNH46686) | 10 | 9 | 1 | 0.72 | 22.78 | 561.50 |

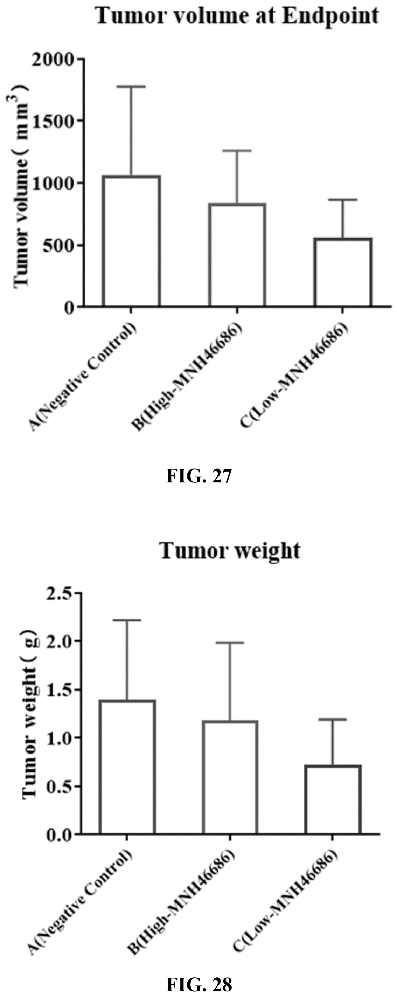

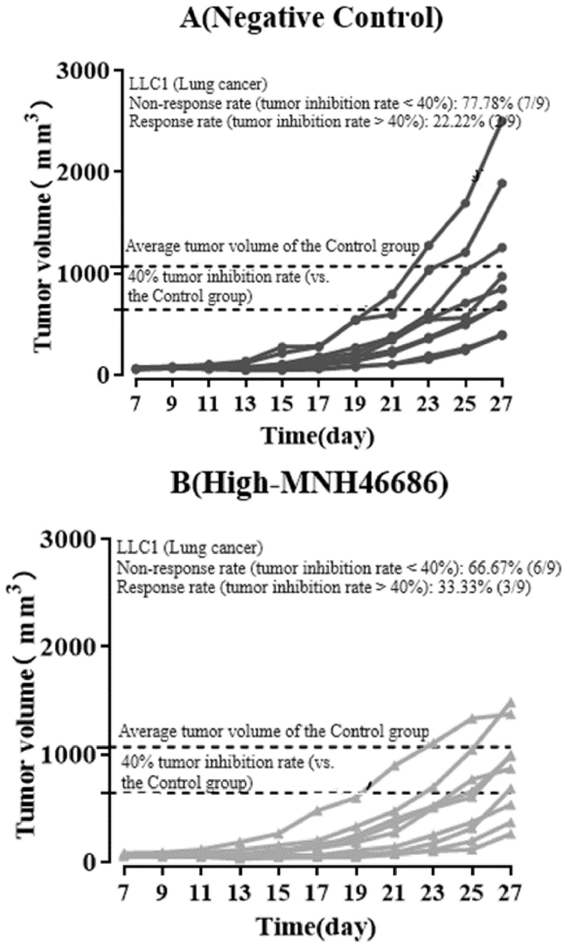

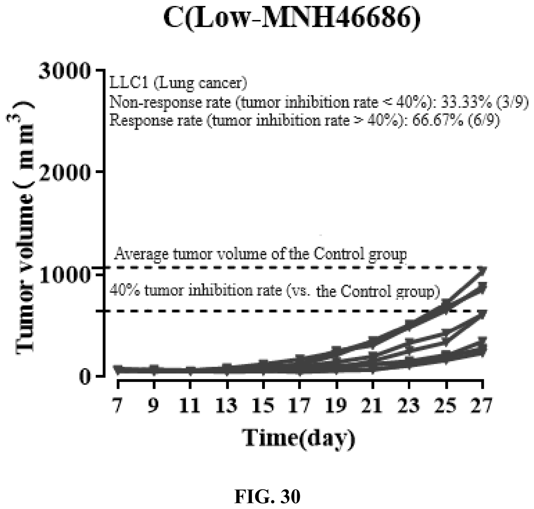

During the experiment, no other abnormalities were found in any mice except for erythema, scabs, tumor cavities, or hypothermia caused by the tumor. At the experimental endpoint, the statistical analysis results of the tumor volume curve of Group C (Low - MNH46686) were significantly smaller than those of Group A (Negative Control), and there was no significant difference between the other groups and Group A (FIG. 26). At the experimental endpoint (D27), the average tumor volumes of Groups A to C were 1067.54 mm³, 838.01 mm³, and 561.50 mm³, respectively, and the average tumor weights were 1.39 g, 1.18 g, and 0.72 g, respectively. The tumor volumes and tumor weights at the experimental endpoint of Groups B to C vs. Group A are shown in FIGS. 27 and 28. At the experimental endpoint, the TGIs of Groups B to C were 23.00% and 50.30%, respectively. The TGIs at the experimental endpoint of Groups B to C vs. Group A are shown in FIG. 30. The response rate of Group A was 22.22%, the response rate of Group B was 33.33%, and the response rate of Group C was 66.67% (FIG. 30).

Finally, it should be noted that the above examples are only used to illustrate but not to limit the technical solutions of the present disclosure. Although the present disclosure has been described in detail with reference to the foregoing examples, those of ordinary skill in the art should understand that the technical solutions described in the foregoing examples can still be modified, or some or all of the technical features can be equivalently substituted. Such modifications or substitutions will not cause the essence of the corresponding technical solutions to deviate from the scope of technical solutions in the examples of the present disclosure.

## Claims

1. Use of a microbial strain of *Lachnospiraceae,* or a culture thereof, or a metabolite thereof, or a fermentation broth thereof, or a supernatant of said fermentation broth in the manufacture of a medicament for preventing and/or treating tumors, **characterized in that** said microbial strain of *Lachnospiraceae* belongs to a new genus and species of the family *Lachnospiraceae* (*Lachnospiraceae sp*.), said microbial strain of *Lachnospiraceae* has a 16S rDNA of the nucleotide sequence as shown in SEQ ID No. 1, or has at least 90% identity thereto, for example, at least 91%, 92%, 93%, 94%, 95%, 96%, 97%, 97.5%, 98%, 98.65%, 98.7%, or 99%, for example, 97% or 98.7% or 99% identity of the 16S rDNA; the tumors include solid tumors, soft tissue tumors, hematopoietic tumors, adenomas, or metastatic tumors; preferably, the tumor is selected from at least one of liver cancer, colon cancer, rectal cancer,colorectal cancer, lung cancer, breast cancer, cervical cancer, ovarian cancer, pancreatic cancer, cholangiocarcinoma, kidney cancer, glioma, liposarcoma, melanoma, lymphoma, small cell lung cancer, squamous cell carcinoma, chondrosarcoma, and fibrosarcoma; or the tumor is a virus-induced tumor or a bacteria-induced tumor, such as a tumor caused by infection with human papillomavirus (HPV), hepatitis B or C virus (HBV, HCV), human immunodeficiency virus (HIV), Epstein-Barr virus, or a tumor caused by infection with Helicobacter pylori (HP) or Fusobacterium nucleatum.

2. The use according to claim 1, wherein the microbial strain of *Lachnospiraceae* comprises *Lachnospiraceae* strain MNH 46686, deposited with the Guangdong Microbial Culture Collection Center, with a deposit name of *Lachnospiraceae sp.* MNH 46686 and a deposit number of GDMCC No: 62002;
or the microbial strain of *Lachnospiraceae* has an average nucleotide identity (ANI) value of at least 95% compared to the *Lachnospiraceae* strain MNH 46686, preferably, the average nucleotide identity ANI value is 95.5%, 96%, 96.5%, 97%, 97.5%, 98%, 98.5%, 99%, 99.5%, 99.6%, 99.7%, 99.8%, 99.9% or 100%.

3. A composition comprising a microbial strain of *Lachnospiraceae,* wherein the microbial strain of *Lachnospiraceae* belongs to a new genus and species of the family *Lachnospiraceae* (*Lachnospiraceae sp*.), said microbial strain of *Lachnospiraceae* has a 16S rDNA of the nucleotide sequence as shown in SEQ ID No. 1, or has at least 90% identity thereto, such as at least 91%, 92%, 93%, 94%, 95%, 96%, 97%, 97.5%, 98%, 98.65%, 98.7%, 99% identity, for example 97% or 98.7% or 99% identity of 16S rDNA ;
preferably, the composition is for oral administration;
preferably, the composition further comprises a pharmaceutically acceptable carrier;
preferably, the microbial strain of *Lachnospiraceae* and the pharmaceutically acceptable carrier are present in an enteric coating.

4. The composition according to claim 3, wherein the microbial strain of *Lachnospiraceae* comprises *Lachnospiraceae* strain MNH 46686, deposited with the Guangdong Microbial Culture Collection Center, with a deposit name of *Lachnospiraceae sp.* MNH 46686 and a deposit number of GDMCC No: 62002;
or the microbial strain of *Lachnospiraceae* has an average nucleotide identity (ANI) value of at least 95% to the *Lachnospiraceae* strain MNH 46686, preferably, the average nucleotide identity ANI value is 95.5%, 96%, 96.5%, 97%, 97.5%, 98%, 98.5%, 99%, 99.5%, 99.6%, 99.7%, 99.8%, 99.9% or 100%.

5. The composition according to any one of claims 3 to 4, wherein the composition comprises live cells of the microbial strain of *Lachnospiraceae,* live bacteria, attenuated bacteria, irradiated bacteria ,inactivated bacteria, a culture of said microbial strain, a metabolite of said microbial strain, a fermentation broth of said microbial strain, or a supernatant of said fermentation broth.

6. The composition according to claim 5, wherein the composition further comprises a co-drug; the co-drug from the group consisting at least one of: a chemotherapeutic agent, a photosensitizing agent, a photothermal agent, an immunotherapeutic agent, and an agent for enhancing cell therapy;
preferably, the chemotherapeutic agent includes at least one of paclitaxel, camptothecin, 5-fluorouracil, cisplatin, doxorubicin, mitomycin or epirubicin;
preferably, the photosensitizing agent includes at least one of boron dipyrrole, chlorin, or Rose Bengal;
preferably, the photothermal agent is one or more of indocyanine green, new indocyanine green, or gold nanoparticle rods;
preferably, the immunotherapeutic agent includes an Anti-PD-1 antibody, CTLA-4 antibody, PD-L1 antibody, or PD-L1 inhibitor;
more preferably, the PD-L1 inhibitor is selected from the group consisting of durvalumab, atezolizumab and avelumab;
the Anti-PD-1 antibody or PD-L1 antibody is selected from the group consisting of pembrolizumab or nivolumab;
the CTLA-4 antibody is selected from the group consisting of ipilimumab;
preferably, the effective dose of the PD-1 inhibitor is 1~11 mg/kg; preferably, the effective dose includes: 1 mg/kg, 2 mg/kg, 3 mg/kg, 4 mg/kg, 5 mg/kg, 6 mg/kg, 7 mg/kg, 8 mg/kg or 9 mg/kg or 10 mg/kg;
preferably, the enhanced cell therapy includes CAR-T;
preferably, the content of the microbial strain in a unit dose of the composition includes 2×10⁷~9×10¹⁰ CFU/mL or 1×10⁶~2×10¹⁰ CFU/mg of the microbial strain of *Lachnospiraceae;* preferably, the unit dose is at least one of 4×10⁷ CFU/mL, 5×10⁷ CFU/mL, 6×10⁷ CFU/mL, 7×10⁷ CFU/mL, 8×10⁷ CFU/mL, 1×10⁸ CFU/mL, 2×10⁸ CFU/mL, 3×10⁸ CFU/mL, 4×10⁸ CFU/mL, 5×10⁸ CFU/mL, 6×10⁸ CFU/mL, 7×10⁸ CFU/mL, 8×10⁸ CFU/mL, 9×10⁸ CFU/mL, 1×10⁹ CFU/mL, 2×10⁹ CFU/mL, 3×10⁹ CFU/mL, 4×10⁹ CFU/mL, 5 ^{x} 10⁹ CFU/mL, 6×10⁹ CFU/mL, 7×10⁹ CFU/mL, 8×10⁹ CFU/mL, 9×10⁹ CFU/mL, 2×10¹⁰ or 1.2×10⁶ CFU/mg, 4×10⁶ CFU/mg, 5×10⁷ CFU/mg, 6×10⁷ CFU/mg, 7×10⁷ CFU/mg, 8×10⁷ CFU/mg, 9×10⁷ CFU/mg, 1×10⁸ CFU/mg, 2×10⁸ CFU/mg, 3×10⁸ CFU/mg, 4×10⁸ CFU/mg, 5×10⁸ CFU/mg, 6×10⁸ CFU/mg, 7×10⁸ CFU/mg, 8×10⁸ CFU/mg, 9×10⁸ CFU/mg.

7. The composition according to claim 5, wherein the composition further comprises a pharmaceutically acceptable carrier; preferably, the carrier is selected from one or more of a diluent, dispersing agent, excipient, stabilizer, lubricant, and disintegrating agent;
preferably, the drug is in the form of a liquid preparation, a solid preparation, a capsule preparation, a sustained-release preparation, and a nano preparation.

8. The composition according to claim 5, wherein at least 50% of the bacteria are live, for example, at least 90% are live bacteria.

9. The composition according to claim 5, wherein the composition is in the form of powder, microencapsulated powder, capsule, tablet, lozenge, granule, emulsion, suspension, suppository, beverage, food, medicine or nutraceutical, food additive, dietary supplement or dairy product.

10. Use of the composition according to any one of claims 3 to 9 in the manufacture of a medicament for preventing and/or treating at least one disease selected from the group consisting of metabolic diseases, diabetes, autoimmune diseases, infectious diseases, central nervous system diseases, and inflammatory bowel diseases.

11. Use of the composition according to any one of claims 3 to 9 in the manufacture of a drug for anti-tumor or tumor growth inhibition;
preferably, inhibiting tumor growth includes at least one of the following: (a) inhibiting tumor volume growth; (b) inhibiting tumor weight increase; (c) inhibiting tumor cell growth; (d) improving tumor treatment response rate; (e) improving the efficacy of immunosuppressive drug treatment, such as improving the efficacy of PD-1 drug treatment; (f) inhibiting tumor cell metastasis; (g) reducing PD-1 resistance; (h) increasing the NK ratio in peripheral blood to activate the subject's systemic immunity; (i) inhibiting HDAC activity through at least one of acetate, propionate, butyrate or valerate in SCFA; (j) inhibiting tumors through at least one of acetate, propionate, butyrate or valerate in SCFA; (k) inhibiting tumors by regulating the subject's immune system to exert an immunomodulatory effect;
the tumor includes solid tumors, soft tissue tumors, hematopoietic tumors, glandular tumors or metastatic tumors; preferably, the tumor is selected from at least one of liver cancer, colon cancer, rectal cancer, colorectal cancer, lung cancer, breast cancer, cervical cancer, ovarian cancer, pancreatic cancer, bile duct cancer, kidney cancer, glioma, liposarcoma, melanoma, lymphoma, small cell lung cancer, squamous cell carcinoma, chondrosarcoma and fibrosarcoma ; or the tumor is a tumor caused by a virus or a tumor caused by bacteria, such as human papillomavirus infection (HPV), hepatitis B or C infection (HBV, HCV), human immunodeficiency virus infection (HIV), Helicobacter pylori infection (HP), Epstein-Barr virus infection , and Helicobacter pylori (HP) infection and Fusobacterium nucleatum (F.nucleatum) infection.

12. Use of the composition according to any one of claims 3 to 9 in the preparation of a medicament for treating and/or preventing a disease mediated by HDAC activity , wherein preferably, the disease mediated by HDAC activity comprises at least one of tumors, metabolic diseases, diabetes, autoimmune diseases, infectious diseases, central nervous system diseases, and inflammatory bowel disease; preferably, the tumor comprises a solid tumor, a soft tissue tumor, a hematopoietic tumor, a glandular tumor, or a metastatic tumor; preferably, the tumor is selected from liver cancer, colon cancer, rectal cancer, colorectal cancer, lung cancer, breast cancer, cervical cancer, ovarian cancer, , pancreatic cancer, bile duct cancer, renal cancer, glioma, liposarcoma, melanoma, lymphoma, small cell lung cancer, squamous cell carcinoma, chondrosarcoma and fibrosarcoma ; or the tumor is a tumor caused by viral infection or a tumor caused by bacterial infection, such as human papillomavirus infection (HPV), hepatitis B or C infection (HBV, HCV), human immunodeficiency virus infection (HIV), Epstein-Barr virus infection, and Helicobacter pylori (HP) infection , Fusobacterium nucleatum (F.nucleatum) infection caused tumors .

13. A microbial strain of *Lachnospiraceae,* or a culture thereof, or a metabolite thereof, or a fermentation broth thereof, or a supernatant of said fermentation broth, **characterized in that** said microbial strain of *Lachnospiraceae* belongs to a new genus and species of the family *Lachnospiraceae* (*Lachnospiraceae sp*.), said microbial strain of *Lachnospiraceae* has a 16S rDNA nucleotide sequence as shown in SEQ ID No. 1.

14. The microbial strain of *Lachnospiraceae* according to claim 13, wherein the microbial strain of *Lachnospiraceae* comprises *Lachnospiraceae* strain MNH 46686, deposited with the Guangdong Microbial Culture Collection Center, with a deposit name of *Lachnospiraceae sp.* MNH 46686 and a deposit number of GDMCC No: 62002.

15. A method for treating or preventing a tumor or diabetes, comprising administering to a subject in need with an effective amount of the microbial strain of *Lachnospiraceae* according to claim 13 or 14,or a culture of the *Lachnospiraceae* microbial strain, or a metabolite of the *Lachnospiraceae* microbial strain, or a fermentation broth of the *Lachnospiraceae* microbial strain, or a supernatant of the fermentation broth, or a composition as claimed in any one of claims 3 to 9;
preferably, the tumor comprises a solid tumor, a soft tissue tumor, a hematopoietic tumor, a glandular tumor or a metastatic tumor; preferably, the tumor is selected from one or more of liver cancer, colon cancer, rectal cancer, colorectal cancer, lung cancer, breast cancer, cervical cancer, ovarian cancer, pancreatic cancer, bile duct cancer, kidney cancer, glioma, liposarcoma, melanoma, lymphoma, small cell lung cancer, squamous cell carcinoma, chondrosarcoma and fibrosarcoma; or the tumor is a tumor caused by a virus or a tumor caused by a bacterium, such as a tumor caused by human papillomavirus infection, hepatitis B infection or hepatitis C infection, human immunodeficiency virus infection, Helicobacter pylori infection, Epstein-Barr virus infection or Fusobacterium nucleatum infection;
preferably, the strain prevents and / or treats tumors by inhibiting tumor growth, and the inhibition of tumor growth includes at least one of the following: ( a ) inhibiting tumor volume growth; ( b ) inhibiting tumor weight increase; ( c ) inhibiting tumor cell growth; ( d ) improving tumor treatment response rate; ( e ) improving the efficacy of immunosuppressive drug treatment, such as improving the efficacy of PD-1 drug treatment; ( f) inhibiting tumor cell metastasis; ( g ) reducing PD-1 resistance; ( h ) increasing the NK ratio in peripheral blood to activate the subject's systemic immunity; ( i ) inhibiting HDAC activity through at least one of acetic acid or acetate , propionic acid or propionate, butyric acid or butyrate, valeric acid or valeric acid in SCFA ; ( j ) inhibiting tumors through at least one short-chain fatty acid or short-chain fatty acid salt of acetic acid or acetate, propionic acid or propionate, butyric acid or butyrate, valeric acid or valeric acid in SCFA; ( k ) inhibiting tumors by regulating the subject's immune system to exert an immunomodulatory effect.

16. The *Lachnospiraceae* microbial strain according to claim 13 or 14, or the culture of the *Lachnospiraceae* microbial strain, or the metabolite of the *Lachnospiraceae* microbial strain, or the fermentation broth of the *Lachnospiraceae* microbial strain, or the supernatant of the fermentation broth, or the composition according to any one of claims 3 to 9, for preventing and / or treating tumors or diabetes;
preferably, the tumor comprises a solid tumor, a soft tissue tumor, a hematopoietic tumor, a glandular tumor or a metastatic tumor; preferably, the tumor is selected from one or more of liver cancer, colon cancer, rectal cancer, colorectal cancer, lung cancer, breast cancer, cervical cancer, ovarian cancer, pancreatic cancer, bile duct cancer, kidney cancer, glioma, liposarcoma, melanoma, lymphoma, small cell lung cancer, squamous cell carcinoma, chondrosarcoma and fibrosarcoma; or the tumor is a tumor caused by a virus or a tumor caused by a bacterium, such as a tumor caused by human papillomavirus infection, hepatitis B infection or hepatitis C infection, human immunodeficiency virus infection, Helicobacter pylori infection, Epstein-Barr virus infection or Fusobacterium nucleatum infection;
preferably, the strain prevents and / or treats tumors by inhibiting tumor growth, and the inhibition of tumor growth includes at least one of the following: ( a ) inhibiting tumor volume growth; ( b ) inhibiting tumor weight increase; ( c ) inhibiting tumor cell growth; ( d ) improving tumor treatment response rate; ( e ) improving the efficacy of immunosuppressive drug treatment, such as improving the efficacy of PD-1 drug treatment; ( f) inhibiting tumor cell metastasis; ( g ) reducing PD-1 resistance; ( h ) increasing the NK ratio in peripheral blood to activate the subject's systemic immunity; ( i ) inhibiting HDAC activity through at least one of acetic acid or acetate , propionic acid or propionate, butyric acid or butyrate, valeric acid or valeric acid in SCFA ; ( j ) inhibiting tumors through at least one short-chain fatty acid or short-chain fatty acid salt of acetic acid or acetate, propionic acid or propionate, butyric acid or butyrate, valeric acid or valeric acid in SCFA; ( k ) inhibiting tumors by regulating the subject's immune system to exert an immunomodulatory effect.
